(19) Europäisches Patentamt
European Patent Office
Office européen des brevets

(11) Publication number : **0 420 464 B1**

(12) # EUROPEAN PATENT SPECIFICATION

(45) Date of publication of patent specification : **13.07.94 Bulletin 94/28**

(51) Int. Cl.$^5$ : **C07D 261/12,** A61K 31/42, C07D 261/20, C07D 413/04

(21) Application number : **90310125.1**

(22) Date of filing : **17.09.90**

(54) **3-Isoxazolone derivatives, their preparation and their therapeutic uses.**

(30) Priority : **26.09.89 JP 249700/89**
**12.04.90 JP 96976/90**

(43) Date of publication of application : **03.04.91 Bulletin 91/14**

(45) Publication of the grant of the patent : **13.07.94 Bulletin 94/28**

(84) Designated Contracting States : **AT BE CH DE DK ES FR GB GR IT LI LU NL SE**

(56) References cited :
**EP-A- 0 273 744**
**CHEMICAL ABSTRACTS, vol. 104, no. 7, 17th February 1986, page 516, abstract no. 50810a, Columbus, Ohio, US; T. SLAWIK: "New acyl and alkyl derivatives of 1,2-benzoisoxazole"**

(73) Proprietor : **Sankyo Company Limited**
**5-1 Nihonbashi Honcho 3-chome**
**Chuo-ku**
**Tokyo (JP)**

(72) Inventor : **Nagano, Mitsuo, c/o Sankyo Company Limited**
**2-58, Hiromachi 1-chome,**
**Shinagawa-ku**
**Tokyo (JP)**
Inventor : **Sakai, Junichi, c/o Sankyo Company Limited**
**2-58, Hiromachi 1-chome,**
**Shinagawa-ku**
**Tokyo (JP)**
Inventor : **Iwata, Nobuyoshi, c/o Sankyo Company Limited**
**2-58, Hiromachi 1-chome,**
**Shinagawa-ku**
**Tokyo (JP)**

(74) Representative : **Gibson, Christian John Robert et al**
**MARKS & CLERK,**
**57/60 Lincoln's Inn Fields**
**London WC2A 3LS (GB)**

EP 0 420 464 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to a series of new 3-isoxazolone derivatives which may be used for the treatment of cerebrovascular disorders, for use as antidepressants and for use as centrally acting muscle relaxants. The invention also provides methods of using these compounds and processes for preparing them.

Cerebrovascular disorders, including stroke infarction, are among the main causes of death in the world today. Moreover, although the patient may survive in such cases, cognition impairment, which may be one of the sequelae of the disease, is extremely distressing to the patients and to those caring for them and is a substantial social problem at present. For this reason, the development of therapeutic agents for the treatment of such disorders (commonly referred to as "cerebro-active drugs") is required.

Furthermore, various diseases associated with senility are rapidly increasing in line with the increase in the average age of the population. One such disease is senile depression, and, indeed, the resulting increase in the incidence of suicide by the aged has become a social problem. Therefore, there has arisen a need to develop therapeutic agents for treating such diseases.

Moreover, rigidity and/or spasticity are often observed as the sequelae of cerebrovascular disorders, such as cerebral stroke infarction, or as cerebrotraumatic sequelae, and make rehabilitation of the patient difficult. Centrally-acting muscle relaxants are available which can relieve such spasrigido hemiplegia, and act on the central nervous system. Examples of centrally-acting muscle relaxants which are currently available include eperisone hydrochloride and afloqualone.

3-Isoxazolone compounds having this type of activity are disclosed in EP 273 744, EP 334 674, EP 335 723 and EP Application No. 90306960. 7, having a priority date earlier than the present application but unpublished at the filing date of this application. None of these prior compounds, however, has the unique ester group of the present invention.

We have now discovered a series of new compounds which can be used as cerebro-active drugs, as antidepressants and as centrally-acting muscle relaxants. Such compounds are needed which can treat cerebral circulatory problems and alleviate myotony and spasrigido hemiplegia, preferably without giving rise to accompanying drowsiness. Moreover, the new isoxazolone derivatives of the present invention can protect the brain from the effects of anoxia.

The compounds of the present invention are those compounds of formula (I):

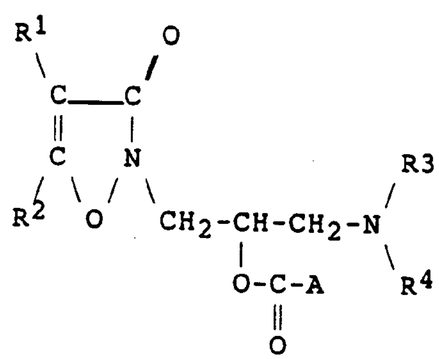

(I)

in which:

$R^1$ represents: a hydrogen atom, a halogen atom; an alkyl group having from 1 to 6 carbon atoms; an alkenyl group having from 2 to 6 carbon atoms; an alkynyl group having from 2 to 6 carbon atoms; an unsubstituted benzyl group; a substituted benzyl group which is substituted by at least one of substituents (a), defined below; an unsubstituted phenyl group; or a substituted phenyl group which is substituted by at least one of substituents (a), defined below;

$R^2$ represents: a hydrogen atom; an alkyl group having from 1 to 6 carbon atoms; an unsubstituted phenyl group; a substituted phenyl group which is substituted by at least one of substituents (a), defined below; or a heterocyclic group having from 5 to 7 ring atoms, of which 1 or 2 are nitrogen and/or oxygen and/or sulphur hetero-atoms, said ring being unsubstituted or being substituted by at least one of substituents (a), defined below, and/or by an oxygen atom; or $R^1$ and $R^2$, together with the carbon atoms to which they are attached, form a hydrocarbon ring having from 5 to 7 ring atoms fused to the isoxazole ring, said hydrocarbon ring being unsubstituted or being substituted by at least one of substituents (a), defined below;

$R^3$ and $R^4$, together with the nitrogen atom to which they are attached, represent an alicyclic amino group having from 5 to 7 ring atoms and having, in addition to the nitrogen atom of the group $-NR^3R^4$, 0 or 1 additional

nitrogen and/or oxygen and/or sulphur hetero-atom, said ring being unsubstituted or being substituted by at least one of substituents (a), defined below, and/or by an oxygen atom; and
A represents: an unsubstituted phenyl group; a substituted phenyl group which is substituted by at least one of substituents (a), defined below; an unsubstituted benzyl group; a substituted benzyl group which is substituted by at least one of substituents (a), defined below; an alkyl group having from 1 to 6 carbon atoms; an alkoxy group having from 1 to 6 carbon atoms; an unsubstituted benzyloxy group; a substituted benzyloxy group which is substituted by at least one of substituents (a), defined below; an unsubstituted phenoxy group; or a substituted phenoxy group which is substituted by at least one of substituents (a), defined below;

substituents (a):

alkyl groups having from 1 to 3 carbon atoms, alkoxy groups having from 1 to 3 carbon atoms, hydroxy groups, halogen atoms, nitro groups, amino groups, aliphatic carboxylic acyl groups having from 2 to 4 carbon atoms and aliphatic carboxylic acylamino groups having from 2 to 4 carbon atoms;
and acid addition salts thereof.

The present invention also provides the use of at least one compound of formula (I) or a pharmaceutically acceptable acid addition salt thereof, as defined above, for the treatment of cerebrovascular disorders, for use as a centrally-acting muscle relaxant or for use as an antidepressant.

The invention also provides the use of at least one compound of formula (I) or a pharmaceutically acceptable acid addition salt thereof, as defined above, for the manufacture of a medicament for the treatment of cerebrovascular disorders, for use as a centrally-acting muscle relaxant or for use as an antidepressant.

The depression may be, for example, senile depression.

The invention also provides a pharmaceutical composition for the treatment of cerebrovascular disorders or as an antidepressant or as a centrally-acting muscle relaxant, which composition comprises at least one compound of formula (I) or a pharmaceutically acceptable acid addition salt thereof, as defined above, in admixture with a pharmaceutically acceptable carrier or diluent.

The invention also provides processes for preparing the compounds of the present invention, which processes are described in more detail hereafter.

In the compounds of the present invention, where $R^1$ represents a halogen atom, this may be, for example, a fluorine, chlorine, bromine or iodine atom, more preferably a fluorine, chlorine or bromine atom, and most preferably a chlorine atom.

Where $R^1$ represents an alkyl group having from 1 to 6 carbon atoms, more preferably from 1 to 4 carbon atoms, this may be a straight or branched chain group, and examples include the methyl, ethyl, propyl, isopropyl, butyl, isobutyl, sec-butyl, pentyl, isopentyl, 2-methylbutyl, hexyl, isohexyl and 2-methylpentyl groups, of which the methyl, ethyl, propyl, isopropyl, butyl, isobutyl, t-butyl, pentyl, isopentyl and hexyl groups are preferred, and the methyl, ethyl, isopropyl and isobutyl groups are more preferred.

Where $R^1$ represents an alkenyl group having from 2 to 6 carbon atoms, this may be a straight or branched chain group, preferably having from 2 to 4 carbon atoms, and examples include the vinyl, allyl, 1-propenyl, 2-propenyl, 1-butenyl, 2-butenyl, 3-butenyl, 3-methylallyl and 2-methylallyl groups, of which the vinyl, allyl, 2-butenyl, 3-methylallyl and 2-methylallyl groups are preferred and the allyl and 3-methylallyl groups are most preferred.

Where $R^1$ represents an alkynyl group having from 2 to 6 carbon atoms, this may be a straight or branched chain group, preferably having from 2 to 4 carbon atoms, and examples include the ethynyl, 1-propynyl, 2-propynyl (i. e. propargyl), 1-butynyl, 2-butynyl and 3-butynyl groups, of which the ethynyl and 2-propynyl groups are most preferred.

Where $R^1$ represents a benzyl group, this may be unsubstituted or it may have one or more substituents. If it is substituted, it may have from 1 to 5 substituents, more preferably from 1 to 3 substituents, and most preferably 1 or 2 substituents, on the benzene ring, and these substituents may be selected from substituents (a), defined above and exemplified in more detail below. Examples of such optionally substituted benzyl groups include the benzyl group itself, and the 2-chlorobenzyl, 3-chlorobenzyl, 4-chlorobenzyl, 2-fluorobenzyl, 3-fluorobenzyl, 4-fluorobenzyl, 2-bromobenzyl, 3-bromobenzyl, 4-bromobenzyl, 2-nitrobenzyl, 3-nitrobenzyl, 4-nitrobenzyl, 2-methoxybenzyl, 3-methoxybenzyl, 4-methoxybenzyl, 2-propoxybenzyl, 3-propoxybenzyl, 4-propoxybenzyl, 2-hydroxybenzyl, 3-hydroxybenzyl, 4-hydroxybenzyl, 2-aminobenzyl, 3-aminobenzyl, 4-aminobenzyl, 2-propylbenzyl, 3-propylbenzyl, 4-propylbenzyl, 2-methylbenzyl, 3-methylbenzyl, 4-methylbenzyl, 2-acetylbenzyl, 3-acetylbenzyl, 4-acetylbenzyl, 2-butyrylbenzyl, 3-butyrylbenzyl, 4-butyrylbenzyl, 2,4-dichlorobenzyl, 2,4-difluorobenzyl, 2-chloro-4-fluorobenzyl, 2-chloro-4-butylbenzyl, 3,5-dichlorobenzyl, 3,5-difluorobenzyl, 3-chloro-5-fluorobenzyl and 3-chloro-5-butylbenzyl groups, but the benzyl group itself is preferred.

Where $R^1$ represents a phenyl group, this may be unsubstituted or it may have one or more substituents.

If it is substituted, it may have from 1 to 5 substituents, more preferably from 1 to 3 substituents, and most preferably 1 or 2 substituents, which may be selected from substituents (a), defined above and exemplified in more detail below. Examples of such optionally substituted phenyl groups include the phenyl group itself, and the 2-chlorophenyl, 3-chlorophenyl, 4-chlorophenyl, 2-fluorophenyl, 3-fluorophenyl, 4-fluorophenyl, 2-bromophenyl, 3-bromophenyl, 4-bromophenyl, 2-nitrophenyl, 3-nitrophenyl, 4-nitrophenyl, 2-methoxyphenyl, 3-methoxyphenyl, 4-methoxyphenyl, 2-propoxyphenyl, 3-propoxyphenyl, 4-propoxyphenyl, 2-hydroxyphenyl, 3-hydroxyphenyl, 4-hydroxyphenyl, 2-aminophenyl, 3-aminophenyl, 4-aminophenyl, 2-propylphenyl, 3-propylphenyl, 4-propylphenyl, 2-methylphenyl, 3-methylphenyl, 4-methylphenyl, 2-acetylphenyl, 3-acetylphenyl, 4-acetylphenyl, 2-butyrylphenyl, 3-butyrylphenyl, 4-butyrylphenyl, 2,4-dichlorophenyl, 2,4-difluorophenyl, 2-chloro-4-fluorophenyl, 2-chloro-4-butylphenyl, 3,5-dichlorophenyl, 3,5-difluorophenyl, 3-chloro-5-fluorophenyl and 3-chloro-5-butylphenyl groups, but the phenyl group itself is preferred.

In general, preferred groups and atoms which may be represented by $R^1$ include the hydrogen and chlorine atoms, and the methyl, ethyl, isopropyl, isobutyl, allyl, 3-methylallyl, 2-propynyl, phenyl and benzyl groups.

Where $R^2$ represents an alkyl group, this may be as defined and exemplified above in relation to the alkyl groups which may be represented by $R^1$. Preferred alkyl groups are the methyl, ethyl, propyl, isopropyl, butyl, isobutyl, sec-butyl and t-butyl groups, of which the methyl group is most preferred.

Where $R^2$ represents a phenyl group, this may be as defined and exemplified above in relation to the optionally substituted phenyl groups which may be represented by $R^1$. Preferred optionally substituted phenyl groups for $R^2$ are the phenyl group itself, and the 4-chlorophenyl, 3-chlorophenyl, 4-nitrophenyl, 4-methoxyphenyl, 3-methoxyphenyl, 2-methoxyphenyl, 4-hydroxyphenyl, 4-fluorophenyl and 2,4-dichlorophenyl groups, of which the phenyl and 4-chlorophenyl groups are more preferred.

Where $R^2$ represents a heterocyclic group, this has from 5 to 7 ring atoms, preferably 5 or 6 ring atoms, of which 1 or 2 are nitrogen and/or oxygen and/or sulphur hetero-atoms, and said heterocyclic group is unsubstituted or has at least one substituent selected from substituents (a), defined above and exemplified in more detail below. The number of such substituents (if any) is not critical and is only limited by the number of substitutable positions and possibly by steric constraints. However, in general, the number of such substituents may be from 1 to 5, although the preferred number is from 1 to 3, more preferably 1 or 2. The heterocyclic group may be aromatic or non-aromatic in character, although it is preferably aromatic. Where there are two hetero-atoms, we prefer that one should be a nitrogen atom and the other should be a nitrogen, oxygen or sulphur atom. Examples of such heterocyclic groups include the thienyl, furyl, pyranyl, pyrrolyl, imidazolyl, pyrazolyl, thiazolyl, isothiazolyl, oxazolyl, isoxazolyl, pyridyl, pyrazinyl, pyrimidinyl, pyridazinyl, furazanyl, pyrrolidinyl, pyrrolinyl, imidazolidinyl, imidazolinyl, pyrazolidinyl, pyrazolinyl, piperidyl, piperazinyl, morpholinyl and thiomorpholinyl groups. Examples of such substituted heterocyclic groups include the 6-chloro-3-pyridyl, 6-(trifluoromethyl)-3-pyridyl, 5-chloro-2-pyridyl, 5-(trifluoromethyl)-2-furyl and 5-(trifluoromethyl)-2-thienyl and 5-chloro-2-thienyl groups. The preferred groups are the thiazolyl, furyl, thienyl and pyridyl groups, of which the thienyl and pyridyl groups, still more preferably the 2-thienyl and 3-pyridyl groups, are most preferred, and these may be unsubstituted or substituted as defined above.

Where $R^1$ and $R^2$, together with the carbon atoms to which they are attached, represent a hydrocarbon ring fused to the isoxazole ring and having, in total (i. e. including the carbon atoms to which $R^1$ and $R^2$ are attached), from 5 to 7 ring carbon atoms, the hydrocarbon ring may be unsubstituted or may have at least one substituent selected from substituents (a), defined above and exemplified in more detail below. Such a hydrocarbon group may be wholly (i. e. aromatically) or partially unsaturated, but is preferably aromatic in character. Examples of 5 to 7 membered ring systems that may be formed by $R^1$ and $R^2$, together with the carbon atoms to which they are attached, include the benzene, cyclohexene, cyclohexadiene, cyclopentene, cyclopentadiene, cycloheptene, cycloheptadiene and cycloheptatriene ring systems, of which the multiply unsaturated groups are preferred, the benzene ring being most preferred. Such ring systems may be unsubstituted or may have at least one substituent selected from substituents (a), defined above and exemplified below. The maximum number of substituents is limited only by the number of substitutable positions on the hydrocarbon ring, although, in the case of "bulky" substituents, steric constraints may also apply. In general, from 1 to 4, more preferably from 1 to 3, substituents are preferred, 1 or 2 being most preferred. Preferred substituted and unsubstituted ring systems are (including the isoxazole ring to which they are attached): the 1,2-benzisoxazole, 5-chloro-1,2-benzisoxazole, 5-amino-1,2-benzisoxazole, 5-acetamido-1,2-benzisoxazole, 5-methoxy-1,2-benzisoxazole, 6-chloro-1,2-benzisoxazole, 4-chloro-1,2-benzisoxazole, 7-chloro-1,2-benzisoxazole, 5-methyl-1,2-benzisoxazole, 5,6-dichloro-1,2-benzisoxazole, 4,5,6,7-tetrachloro-1,2-benzisoxazole, 5-propyl-1,2-benzisoxazole, 5-ethoxy-1,2-benzisoxazole, 5-phenyl-1,2-benzisoxazole, 5-benzyl-1,2-benzisoxazole, 5-(4-chlorophenyl)-1,2-benzisoxazole and 5-(4-chlorobenzyl)-1,2-benzisoxazole groups. Of these, we prefer the 1,2-benzisoxazole, 5-chloro-1,2-benzisoxazole, 5-amino-1,2-benzisoxazole, 5-acetamido-1,2-benzisoxazole, 5-methoxy-1,2-benzisoxazole and 6-chloro-1,2-benzisoxazole groups.

$R^3$, $R^4$ and the nitrogen atom to which they are attached together represent an alicyclic amino group having a total of from 5 to 7, preferably 5 or 6, ring atoms, of which one is said nitrogen atom and 0 or 1 is an additional hetero-atom selected from nitrogen, oxygen and sulphur hetero-atoms, said alicyclic amino group being unsubstituted or substituted. Where there is an additional nitrogen hetero-atom, and the group is substituted, the substituent is preferably on that additional nitrogen hetero-atom. The substituents are selected from oxygen atoms and substituents (a), defined above and exemplified in more detail below. Examples of such groups include the 1-pyrrolyl, 1-imidazolyl, 1-pyrazolyl, 3-thiazolyl, 2-isothiazolyl, 3-oxazolyl, 2-isoxazolyl, 1-pyridyl, 1-pyrazinyl, 1-pyrimidinyl, 1-pyridazinyl, 2-furazanyl, 1-pyrrolidinyl, 1-pyrrolinyl, 1-imidazolidinyl, 1-imidazolinyl, 1-pyrazolidinyl, 1-pyrazolinyl, piperidino, 1-piperazinyl, morpholino and thiomorpholino groups, which may be substituted or unsubstituted, and, if substituted, the substituents are selected from oxygen atoms and substituents (a), defined above and exemplified below. Of these heterocyclic groups, the morpholino, 1-piperazinyl, 4-methyl-1-piperazinyl, 1-pyrrolidinyl and piperidino groups are most preferred.

Examples of groups and atoms included in substituents (a) include:

alkyl groups having from 1 to 3 carbon atoms, such as the methyl, ethyl, propyl and isopropyl groups;
alkoxy groups having from 1 to 3 carbon atoms, such as the methoxy, ethoxy, propoxy and isopropoxy groups;
the hydroxy, nitro and amino groups;
halogen atoms, such as the fluorine, chlorine, bromine and iodine atoms;
aliphatic carboxylic acyl groups having from 2 to 4 carbon atoms, especially the alkanoyl groups having from 2 to 4 carbon atoms, such as the acetyl, propionyl, butyryl, isobutyryl, acryloyl, propioloyl, methacryloyl, crotonoyl and isocrotonoyl groups; and
aliphatic carboxylic acylamino groups having from 2 to 4 carbon atoms, especially the alkanoylamino groups having from 2 to 4 carbon atoms, such as the acetamido, propionamido, butyramido, isobutyramido, acryloylamino, propioloylamino, methacryloylamino, crotonoylamino and isocrotonoylamino groups.

Examples of preferred classes of compounds of the present invention include those compounds of formula (I), in which:

(A) $R^1$ represents a hydrogen atom, a halogen atom or an alkyl group having from 1 to 4 carbon atoms;

(B) $R^2$ represents a phenyl group or a phenyl group having at least one substituent selected from alkoxy groups having from 1 to 3 carbon atoms, hydroxy groups and halogen atoms;

(C) $R^1$ and $R^2$, together with the carbon atoms to which they are attached, represent a 6-membered hydrocarbon ring fused to the isoxazole ring and being unsubstituted or having at least one substituent selected from halogen atoms;

(D) $-NR^3R^4$ represents an alicyclic amino group having a total of 5 or 6 ring atoms, of which one is said nitrogen atom and 0 or 1 is an additional nitrogen hetero-atom, said alicyclic amino group being unsubstituted or, where there is an additional nitrogen hetero-atom, having at least one substituent on that additional nitrogen hetero-atom selected from alkyl groups having from 1 to 3 carbon atoms, benzyl groups and phenyl groups;

(E) A represents an alkyl group having from 1 to 4 carbon atoms, an alkoxy group having from 1 to 4 carbon atoms, a phenyl group, a phenoxy group, a benzyl group or a benzyloxy group, said phenyl, phenoxy, benzyl and benzyloxy groups being unsubstituted or having at least one substituent selected from methoxy groups and halogen atoms;

and pharmaceutically acceptable salts thereof.

More preferred compounds of the present invention are those compounds of formula (I) in which:

(F) $R^2$ represents a phenyl group or a phenyl group having at least one substituent selected from methoxy groups and halogen atoms;

(G) $R^1$ and $R^2$, together with the carbon atoms to which they are attached, represent a benzene ring fused to the isoxazole ring and being unsubstituted or having one substituent selected from halogen atoms;

(H) $-NR^3R^4$ represents a morpholino group, a piperazinyl group (preferably a 1-piperazinyl group), a methyl-substituted piperazinyl group (preferably a 4-methyl-1-piperazinyl group), a 1-pyrrolidinyl group or a piperidyl group (preferably a piperidino group);

(I) A represents an alkyl group having from 1 to 4 carbon atoms, preferably a methyl group, an alkoxy group having from 1 to 4 carbon atoms, preferably a methoxy group, or an unsubstituted phenyl, phenoxy, benzyl or benzyloxy group;

and pharmaceutically acceptable salts thereof.

Still more preferred compounds are those compounds of formula (I), in which:

$R^1$ represents a hydrogen atom, a halogen atom or an alkyl group having from 1 to 4 carbon atoms;
$R^2$ represents a phenyl group or a phenyl group having a halogen substituent;
$-NR^3R^4$ represents a morpholino, piperidyl or piperazinyl group; and
A represents an alkyl or alkoxy group having from 1 to 4 carbon atoms;

and pharmaceutically acceptable salts thereof.

The most preferred compounds of the present invention are those compounds of formula (I), in which:

$R^1$ represents a hydrogen atom, a halogen atom or an alkyl group having from 1 to 4 carbon atoms, especially those in which $R^1$ represents a hydrogen atom, a chlorine atom or a methyl or ethyl group;

$R^2$ represents a phenyl group or a phenyl group having a single halogen substituent;

$-NR^3R^4$ represents a morpholino group; and

A represents an alkoxy group having from 1 to 4 carbon atoms;

and pharmaceutically acceptable salts thereof.

Examples of specific compounds of the invention are given in the following formulae (I-1) and (I-2), in which the substituents are as defined in the corresponding one of Tables 1 and 2, respectively, i.e. Table 1 relates to formula (I-1) and Table 2 relates to formula (I-2).

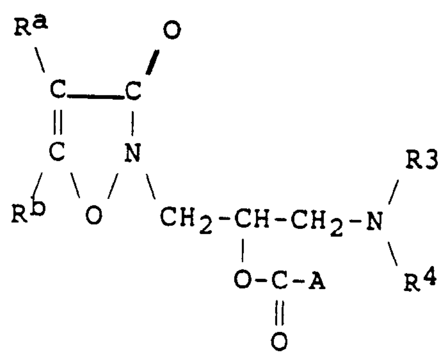

(I-1)

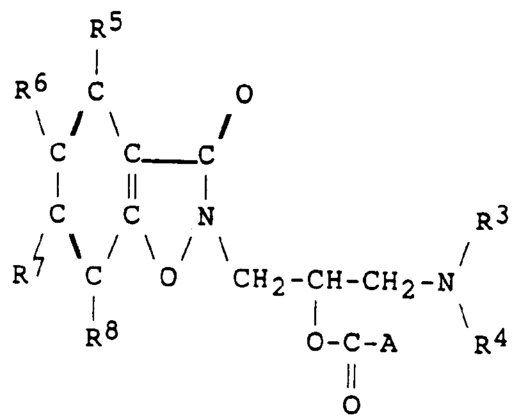

(I-2)

In the following Tables, certain abbreviations are used for the sake of brevity, and these abbreviations have the following meanings:

| | |
|---|---|
| All | allyl |
| Bu | butyl |
| iBu | isobutyl |
| sBu | sec-butyl |
| tBu | t-butyl |
| Bz | benzyl |
| Et | ethyl |
| Me | methyl |
| Mor | morpholino |
| Ph | phenyl |
| Pip | piperidyl |
| Piz | 1-piperazinyl |
| Pr | propyl |
| iPr | isopropyl |
| Prg | propargyl (= 2-propynyl) |
| Pyr | pyridyl |

| | |
|---|---|
| Pyrd | 1-pyrrolidinyl |
| Thi | thienyl |

Table 1

| Cpd. No. | $R^a$ | $R^b$ | $-NR^3R^4$ | A |
|---|---|---|---|---|
| 1-1 | H | Ph | Mor | Me |
| 1-2 | H | Ph | Mor | Et |
| 1-3 | H | Ph | Mor | Pr |
| 1-4 | H | Ph | Mor | iPr |
| 1-5 | H | Ph | Mor | Bu |
| 1-6 | H | Ph | Mor | iBu |
| 1-7 | H | Ph | Mor | sBu |
| 1-8 | H | Ph | Mor | tBu |
| 1-9 | H | Ph | Mor | Ph |
| 1-10 | H | Ph | Mor | Bz |
| 1-11 | Cl | Ph | Mor | Me |
| 1-12 | Cl | Ph | Mor | Et |
| 1-13 | Cl | Ph | Mor | Pr |
| 1-14 | Cl | Ph | Mor | iPr |
| 1-15 | Cl | Ph | Mor | Bu |
| 1-16 | Cl | Ph | Mor | iBu |
| 1-17 | Cl | Ph | Mor | sBu |
| 1-18 | Cl | Ph | Mor | tBu |
| 1-19 | Cl | Ph | Mor | Ph |
| 1-20 | Cl | Ph | Mor | Bz |
| 1-21 | H | p-ClPh | Mor | Me |
| 1-22 | H | p-ClPh | Mor | Et |
| 1-23 | H | p-ClPh | Mor | Pr |
| 1-24 | H | p-ClPh | Mor | iPr |
| 1-25 | H | p-ClPh | Mor | Bu |
| 1-26 | H | p-ClPh | Mor | iBu |
| 1-27 | H | p-ClPh | Mor | sBu |
| 1-28 | H | p-ClPh | Mor | Ph |

Table 1 (cont)

| Cpd. No. | $R^a$ | $R^b$ | $-NR^3R^4$ | A |
|---|---|---|---|---|
| 1-29 | H | p-CℓPh | Mor | Bz |
| 1-30 | H | p-CℓPh | Pyrd | Me |
| 1-31 | H | Ph | Pyrd | Me |
| 1-32 | Cℓ | Ph | Pyrd | Me |
| 1-33 | Cℓ | Ph | Piz | Me |
| 1-34 | H | Ph | Piz | Me |
| 1-35 | H | p-CℓPh | Piz | Me |
| 1-36 | H | p-CℓPh | 4-MePiz | Me |
| 1-37 | H | Ph | 4-MePiz | Me |
| 1-38 | Cℓ | Ph | 4-MePiz | Me |
| 1-39 | Cℓ | Ph | Pip | Me |
| 1-40 | H | Ph | Pip | Me |
| 1-41 | Cℓ | 4-CℓPh | Pip | Me |
| 1-42 | Me | Ph | Mor | Me |
| 1-43 | Et | Ph | Mor | Me |
| 1-44 | iPr | Ph | Mor | Me |
| 1-45 | iBu | Ph | Mor | Me |
| 1-46 | All | Ph | Mor | Me |
| 1-47 | $MeCH=CHCH_2$ | Ph | Mor | Me |
| 1-48 | Prg | Ph | Mor | Me |
| 1-49 | Ph | Ph | Mor | Me |
| 1-50 | Bz | Ph | Mor | Me |
| 1-51 | H | Ph | Mor | OMe |
| 1-52 | H | Ph | Mor | OEt |
| 1-53 | H | Ph | Mor | OPr |
| 1-54 | H | Ph | Mor | OiPr |
| 1-55 | H | Ph | Mor | OBu |
| 1-56 | H | Ph | Mor | OiBu |

Table 1 (cont)

| Cpd. No. | $R^a$ | $R^b$ | $-NR^3R^4$ | A |
|---|---|---|---|---|
| 1-57 | H | Ph | Mor | OsBu |
| 1-58 | H | Ph | Mor | OPh |
| 1-59 | H | Ph | Mor | OBz |
| 1-60 | Cl | Ph | Mor | OMe |
| 1-61 | Cl | Ph | Mor | OEt |
| 1-62 | Cl | Ph | Mor | OPr |
| 1-63 | Cl | Ph | Mor | OiPr |
| 1-64 | Cl | Ph | Mor | OBu |
| 1-65 | Cl | Ph | Mor | OiBu |
| 1-66 | Cl | Ph | Mor | OsBu |
| 1-67 | Cl | Ph | Mor | OtBu |
| 1-68 | Cl | Ph | Mor | OPh |
| 1-69 | Cl | Ph | Mor | OBz |
| 1-70 | Cl | 4-ClPh | Mor | OMe |
| 1-71 | H | 4-ClPh | Mor | OsBu |
| 1-72 | H | 4-ClPh | Mor | OtBu |
| 1-73 | H | 4-ClPh | Mor | OPh |
| 1-74 | H | 4-ClPh | Mor | OBz |
| 1-75 | H | 4-ClPh | Pyrd | OMe |
| 1-76 | H | Ph | Pyrd | OMe |
| 1-77 | Cl | Ph | Pyrd | OMe |
| 1-78 | Cl | Ph | Piz | OMe |
| 1-79 | H | Ph | Piz | OMe |
| 1-80 | H | 4-ClPh | Piz | OMe |
| 1-81 | H | 4-ClPh | Pip | OMe |
| 1-82 | H | Ph | Pip | OMe |
| 1-83 | Cl | Ph | Pip | OMe |
| 1-84 | Me | Ph | Mor | OMe |

Table 1 (cont)

| Cpd. No. | $R^a$ | $R^b$ | $-NR^3R^4$ | A |
|---|---|---|---|---|
| 1-85 | Et | Ph | Mor | OMe |
| 1-86 | iPr | Ph | Mor | OMe |
| 1-87 | iBu | Ph | Mor | OMe |
| 1-88 | All | Ph | Mor | OMe |
| 1-89 | $MeCH=CHCH_2$ | Ph | Mor | OMe |
| 1-90 | Prg | Ph | Mor | OMe |
| 1-91 | Ph | Ph | Mor | OMe |
| 1-92 | Bz | Ph | Mor | OMe |
| 1-93 | H | Me | Mor | OMe |
| 1-94 | H | Et | Mor | OMe |
| 1-95 | H | Pr | Mor | OMe |
| 1-96 | H | iPr | Mor | OMe |
| 1-97 | H | Bu | Mor | OMe |
| 1-98 | H | iBu | Mor | OMe |
| 1-99 | H | sBu | Mor | OMe |
| 1-100 | H | tBu | Mor | OMe |
| 1-101 | H | 4-CℓPh | Mor | OMe |
| 1-102 | H | 3-CℓPh | Mor | OMe |
| 1-103 | H | 2-CℓPh | Mor | OMe |
| 1-104 | H | $4-NO_2Ph$ | Mor | OMe |
| 1-105 | H | 4-MeOPh | Mor | OMe |
| 1-106 | H | 3-MeOPh | Mor | OMe |
| 1-107 | H | 2-MeOPh | Mor | OMe |
| 1-108 | H | 4-HOPh | Mor | OMe |
| 1-109 | H | 4-FPh | Mor | OMe |
| 1-110 | H | 3,4-diCℓPh | Mor | OMe |
| 1-111 | H | 2-Thi | Mor | OMe |
| 1-112 | H | 3-Pyr | Mor | OMe |

Table 2

| Cpd. No. | $R^5$ | $R^6$ | $R^7$ | $R^8$ | $-NR^3R^4$ | A |
|---|---|---|---|---|---|---|
| 2-1 | H | H | H | H | Mor | Me |
| 2-2 | H | H | H | H | Mor | Et |
| 2-3 | H | H | H | H | Mor | Pr |
| 2-4 | H | H | H | H | Mor | iPr |
| 2-5 | H | H | H | H | Mor | Bu |
| 2-6 | H | H | H | H | Mor | iBu |
| 2-7 | H | H | H | H | Mor | sBu |
| 2-8 | H | H | H | H | Mor | tBu |
| 2-9 | H | H | H | H | Mor | Ph |
| 2-10 | H | H | H | H | Mor | Bz |
| 2-11 | H | Cℓ | H | H | Mor | Me |
| 2-12 | H | Cℓ | H | H | Mor | Et |
| 2-13 | H | Cℓ | H | H | Mor | Pr |
| 2-14 | H | Cℓ | H | H | Mor | iPr |
| 2-15 | H | Cℓ | H | H | Mor | Bu |
| 2-16 | H | Cℓ | H | H | Mor | iBu |
| 2-17 | H | Cℓ | H | H | Mor | sBu |
| 2-18 | H | Cℓ | H | H | Mor | tBu |
| 2-19 | H | Cℓ | H | H | Mor | Ph |
| 2-20 | H | Cℓ | H | H | Mor | Bz |
| 2-21 | H | H | H | H | Mor | OMe |
| 2-22 | H | H | H | H | Mor | OEt |
| 2-23 | H | H | H | H | Mor | OPr |
| 2-24 | H | H | H | H | Mor | OiPr |
| 2-25 | H | H | H | H | Mor | OBu |
| 2-26 | H | H | H | H | Mor | OiBu |
| 2-27 | H | H | H | H | Mor | OsBu |
| 2-28 | H | H | H | H | Mor | OtBu |

Table 2 (cont)

| Cpd. No. | $R^5$ | $R^6$ | $R^7$ | $R^8$ | $-NR^3R^4$ | A |
|---|---|---|---|---|---|---|
| 2-29 | H | H | H | H | Mor | OPh |
| 2-30 | H | H | H | H | Mor | OBz |
| 2-31 | H | Cℓ | H | H | Mor | OMe |
| 2-32 | H | Cℓ | H | H | Mor | OEt |
| 2-33 | H | Cℓ | H | H | Mor | OPr |
| 2-34 | H | Cℓ | H | H | Mor | O*i*Pr |
| 2-35 | H | Cℓ | H | H | Mor | OBu |
| 2-36 | H | Cℓ | H | H | Mor | O*i*Bu |
| 2-37 | H | Cℓ | H | H | Mor | O*s*Bu |
| 2-38 | H | Cℓ | H | H | Mor | O*t*Bu |
| 2-39 | H | Cℓ | H | H | Mor | OPh |
| 2-40 | H | Cℓ | H | H | Mor | OBz |
| 2-41 | H | Cℓ | H | H | Pyrd | OMe |
| 2-42 | H | H | H | H | Pyrd | OMe |
| 2-43 | H | H | H | H | Piz | OMe |
| 2-44 | H | Cℓ | H | H | Piz | OMe |
| 2-45 | H | Cℓ | H | H | 4-MePiz | OMe |
| 2-46 | H | H | H | H | 4-MePiz | OMe |
| 2-47 | H | H | H | H | Pip | OMe |
| 2-48 | H | Cℓ | H | H | Pip | Me |
| 2-49 | H | Cℓ | H | H | Pyrd | Me |
| 2-50 | H | H | H | H | Pyrd | Me |
| 2-51 | H | H | H | H | Piz | Me |
| 2-52 | H | Cℓ | H | H | Piz | Me |
| 2-53 | H | Cℓ | H | H | 4-MePiz | Me |
| 2-54 | H | H | H | H | 4-MePiz | Me |

Of the compounds listed above, the following are preferred, that is to say Compounds No. 1-1, 1-2, 1-3, 1-4, 1-5, 1-6, 1-7, 1-8, 1-11, 1-12, 1-13, 1-14, 1-15, 1-16, 1-17, 1-18, 1-21, 1-22, 1-23, 1-24, 1-25, 1-26, 1-27, 1-30, 1-31, 1-32, 1-33, 1-34, 1-35, 1-36, 1-37, 1-38, 1-39, 1-40, 1-41, 1-42, 1-43, 1-44, 1-45, 1-51, 1-52, 1-53, 1-54, 1-55, 1-56, 1-57, 1-60, 1-61, 1-62, 1-63, 1-64, 1-65, 1-66, 1-67, 1-70, 1-71, 1-72, 1-75, 1-76, 1-77, 1-78, 1-79, 1-80, 1-81, 1-82, 1-83, 1-84, 1-85, 1-86, 1-87, 1-93, 1-94, 1-95, 1-96, 1-97, 1-98, 1-99, 1-100, 1-101, 1-102, 1-103, 1-109 and 1-110, and the following are more preferred, that is to say Compounds No. 1-51, 1-52, 1-53, 1-54, 1-55, 1-56, 1-57, 1-60, 1-61, 1-62, 1-63, 1-64, 1-65, 1-66, 1-67, 1-70, 1-71, 1-72, 1-75, 1-76, 1-77, 1-78, 1-79, 1-80, 1-81, 1-82, 1-83, 1-84, 1-85, 1-86, 1-87, 1-93, 1-94, 1-95, 1-96, 1-97, 1-98, 1-99, 1-100, 1-101, 1-102, 1-103, 1-109 and 1-110.

The following compounds are the most preferred, that is to say Compounds No.:

1-21. 2-(2-acetoxy-3-morpholinopropyl)-5-(p-chlorophenyl)-3-isoxazolone;
1-22. 5-(p-chlorophenyl)-2-(2-propionyloxy-3-morpholinopropyl)-3-isoxazolone;
1-51. 2-(2-methoxycarbonyloxy-3-morpholinopropyl)-5-phenyl-3-isoxazolone;
1-52. 2-(2-ethoxycarbonyloxy-3-morpholinopropyl)-5-phenyl-3-isoxazolone;
1-60. 4-chloro-2-(2-methoxycarbonyloxy-3-morpholinopropyl)-5-phenyl-3-isoxazolone;
1-61. 4-chloro-2-(2-ethoxycarbonyloxy-3-morpholinopropyl)-5-phenyl-3-isoxazolone;
1-84. 2-(2-methoxycarbonyloxy-3-morpholinopropyl)-4-methyl-5-phenyl-3-isoxazolone;
1-85. 4-ethyl-2-(2-methoxycarbonyloxy-3-morpholinopropyl)-5-phenyl-3-isoxazolone;
1-109. 5-(p-fluorophenyl)-2-(2-methoxycarboxy-3-morpholinopropyl)-3-isoxazolone;

and pharmaceutically acceptable salts thereof.

The compounds of the present invention can be prepared by a variety of methods well known for the preparation of compounds of this type. In general terms, they may be prepared by the esterification of a compound of formula (II):

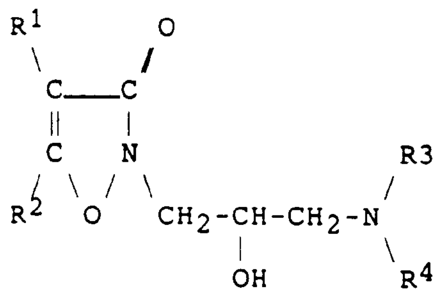

(II)

(in which $R^1$, $R^2$ and $-NR^3R^4$ are as defined above), or such a compound in which any active groups other than the hydroxy group to be esterified are protected, with a carboxylic acid of formula (III):

HO-CO-A (III)

(in which A is as defined above) or with a reactive derivative thereof, preferably the halide of formula (IV):

X-CO-A (III)

(in which A is as defined above and X represents a halogen atom). If there are protecting groups, these may, if necessary, then be removed by conventional means.

Such a reaction is a conventional esterification reaction and may be carried out by conventional, procedures, such as those described in Japanese Patent Application Kokai (i.e. as laid open to public inspection) Sho. No. 56-34674.

Where, as is preferred, the halide of formula (IV) is employed, the halogen represented by X is preferably chlorine or bromine. The nature of the acyl radical A-CO- will, of course, depend on the nature of the ester group which it is desired to introduce, but examples of such halides include: organic acid halides, such as acetyl chloride or benzoyl chloride; and haloformic acid esters, such as methyl chloroformate.

The reaction is preferably carried out in the presence of a base, the nature of which is not critical, provided that it does not adversely affect any other part of the molecule. Examples of suitable bases include: alkali metal hydrides, such as sodium hydride; and organic bases, such as pyridine, triethylamine, N,N-dimethylaniline, 4-dimethylaminopyridine, 1,5-diazabicyclo[4.3.0]non-5-ene (DBN) or 1,8-diazabicyclo[5.4.0]undec-7-ene (DBU). If desired, this organic base may be employed as the reaction solvent. Otherwise, there is no particular restriction on the nature of the solvent to be employed, provided that it has no adverse effect on the reaction or on the reagents involved. Examples of suitable solvents include: ethers, such as diethyl ether, tetrahydrofuran and dioxane; aromatic hydrocarbons, such as benzene and toluene; ketones, such as acetone and methyl butyl ketone; and halogenated hydrocarbons, especially halogenated aliphatic hydrocarbons, such as methylene chloride, chloroform or tetrachloroethane.

The reaction can take place over a wide range of temperatures, and the precise reaction temperature is not critical to the invention. In general, we find it convenient to carry out the reaction at a temperature of from -50°C to 50°C, more preferably at from 0°C to room temperature. The time required for the reaction may also vary widely, depending on many factors, notably the reaction temperature and the nature of the reagents. However, provided that the reaction is effected under the preferred conditions outlined above, a period of from 30

minutes to 4 hours will usually suffice.

After completion of the reaction, the desired compound of formula (I) can be recovered by conventional means from the reaction mixture. For example, the compound can be obtained by filtration, if it separates from the reaction mixture; or, if it is a liquid, it can be obtained by distilling off the solvent, dissolving the residue in a water-immiscible solvent, washing the resulting solution with an acid and water and finally distilling off the solvent. If necessary, the compound can be further purified by such conventional means as recrystallization, vacuum distillation or the various chromatography techniques, notably column chromatography or preparative thin layer chromatography.

The compound of the present invention obtained as described above can be, if desired, converted into a pharmaceutically acceptable acid addition salt. There is no limitation upon the nature of such salts, provided that, where they are to be used for therapeutic purposes, they are pharmaceutically acceptable, which, as is well-known in the art, means that they do not have reduced activity (or unacceptably reduced activity) or increased toxicity (or unacceptably increased toxicity) compared with the free compound of formula (I). Where, however, they are to be used for non-therapeutic purposes, e.g. as intermediates in the preparation of other compounds, even this limitation does not apply. Examples of suitable salts include: salts with an inorganic acid, such as a hydrogen halide (e.g. hydrofluoric acid, hydrochloric acid, hydrobromic acid or hydroiodic acid), or nitric acid, perchloric acid, sulphuric acid or phosphoric acid; and salts with an organic acid, such as a lower alkylsulphonic acid (e.g. methanesulphonic acid, trifluoromethanesulphonic acid or ethanesulphonic acid), an arylsulphonic acid (e.g. benzenesulphonic acid or p-toluenesulphonic acid), or a carboxylic acid (e.g. acetic acid, fumaric acid, lactic acid, citric acid, tartaric acid, succinic acid, oxalic acid or maleic acid). Such salts may be prepared by conventional means such as are well known in the art.

The compounds of the present invention can exist in the form of various optical isomers and diastereomers because of the existence of asymmetric carbon atoms in the molecule. The optical isomers can be resolved using conventional techniques of optical resolution to give optically active compounds. The present invention covers both the individual isomers and mixtures (e.g. racemic mixtures) thereof, whether as obtained by their synthesis reaction or by mixing. If individual isomers are required, these may be prepared from mixtures, by conventional means, or they may be prepared by stereospecific synthesis techniques, as are well known in the art.

## BIOLOGICAL ACTIVITY

As shown in the pharmacological activity and toxicity tests described hereafter, the compounds of the present invention have shown an excellent capacity to counter some of the symptoms elicited by cerebral ischemia, have an antidepressant effect and have a centrally-acting muscle relaxant activity, combined with a low toxicity. Moreover, the compounds of the present invention do not induce much drowsiness, which makes their administration less difficult than that of other similar drugs and is likely to make them much more readily useable.

## EXPERIMENT 1

### Suppression of decerebrate rigidity in rats

The suppression of decerebrate rigidity in laboratory animals, such as rats and cats, is a well known model for the control of human spasticity and/or rigidity.

In the test method, a rat was anesthetized with ether and fixed on a stereotaxic apparatus (SR-5, Narishige). According to the brain atlas by Pellegrino et al., [L. J. Pellegrino, A. S. Pellegrino & A. J. Cushman : A Stereotaxic Atlas of the Rat Brain, Plenum Press, New York and London (1987)], an electrode 0.7 mm in diameter, which was insulated except for 1 mm at the tip, was inserted bilaterally into the midbrain reticular formation (AP: 0, L: ±1.5, H: -3.0). A clip serving as the reference electrode was fixed on the temporal muscle. Through these electrodes, a high frequency electric current (100 KHz, 25 mA) was applied from a lesion generator (Radionics Ltd., RFG-4) for 3 minutes to cauterize electrically this region of the brain.

The rat was then set free from the stereotaxic apparatus. A polyethylene cannula was inserted into the duodenum and fixed with an acrylic resin (Aron Alfa, Sankyo Co., Ltd. ). After completion of these operations, anesthesia was discontinued.

After the animal had woken from the anesthesia, the hind limbs of the rat were fixed on an apparatus devised in our laboratory for hind leg fixation. Both hind legs were fixed at the anterior part of the ankle joints. Both hindlimb paws were then pushed in at the rate of 4 mm for 6 seconds in every minute. The resulting repulsive tension caused by the stretch reflex of the hind limb ankle extensors was recorded on a polygraph

through a FD pick-up (Nihon-Koden). After completion of the experiment, the animals were sacrificed by administration of ether; the passive tension at that time was subtracted from the total tension recorded in the experiment to calculate the active tension.

The test compounds were employed in suspension in a 0.5% w/v CMC (carboxymethyl cellulose) aqueous solution and were administered through the previously inserted cannula.

The results are summarized in the following Table 3; in addition to the compounds of the invention, to provide a basis for comparison, the experiment was also conducted using the known compound, eperisone hydrochloride.

The compounds of the invention are identified in this and subsequent Tables by the number of the one of the following Examples in which they were prepared.

In the Table below, the maximum inhibition rate was calculated from the following equation:

$$\text{Maximum inhibition rate} = 100 - \frac{\text{Active tension before administration of test compound}}{\text{Minimum active tension after administration}}$$

Table 3

Inhibition of decerebrate rigidity in rats

| Cpd. of Ex. No. | Dose (mg/kg) | Route of Administration | Maximum inhibition (%) | Duration of the effect |
|---|---|---|---|---|
| 1 | 50 | i.p. | 85 | >2 hours |
| 2 | 50 | p.o. | 85 | >2 hours |
| 3* | 50 | p.o. | 80 | >1.5 hours |
| eperisone hydrochloride | 50 | p.o. | 50 | 0.5 hour |

* as the hydrochloride.

EXPERIMENT 2

Anti-reserpine effect

It is considered that the sedation caused by the administration of reserpine or tetrabenazine to mice or rats produces an effect similar to the symptoms of clinical depression. Of the various pharmacological effects caused by these compounds, ptosis (drooping of the upper eyelid) and a reduction in body temperature have been widely used as indices of their activity, and it has been shown that any compound which inhibits either ptosis or lowering of the body temperature will also be active against the symptoms of clinical depression. Therefore, anti-reserpine or anti-tetrabenazine effects have been widely used for a long time as a method of screening for antidepressants. The test index used for the evaluation of the compounds of the present invention is the antagonistic effect of a test compound against reserpine-induced ptosis in mice.

The test animals employed were mature male mice of the ddY strain, 4 weeks old and each weighing 22 to 27 g. The animals were divided into groups, each including 3 mice. The compound under test was dissolved or suspended in an appropriate solvent [either physiological saline or a 0.5% CMC (carboxymethyl cellulose)

solution] and administered orally to the mice of the test groups in the dose specified in the following Table 4. The mice in a control group were given only the solvent without any active compound, administered in a similar way. Immediately after administration, 2 mg/kg of reserpine were administered subcutaneously to every mouse. After 90 minutes, it was determined how much the eyelids had closed (degree of ptosis) by bringing the mouse out of its cage and observing the shape of the eye. For assessment of the results, normal mice without ptosis and, hence, with a normal circular eye were scored as 0, while a mouse which exhibited ptosis by 1/3 to 1/2 was scored as 1, a mouse which exhibited ptosis by 2/3 to slightly opened eyes was scored as 2, and a mouse with completely closed eyelids was scored as 3.

The results are shown in Table 4.

The labels of the bottles containing the sample solutions to be tested were all indicated by codes unknown to those responsible for administration, and the administration was carried out in a random order so that the scorers did not know what drug was administered to what mouse. From the scores obtained, the inhibition rate at each dose level was calculated from the following equation:

$$\text{Inhibition rate (\%)} = \left(1 - \frac{\text{Total score of the sample group}}{\text{Total score of the control group}}\right) \times 100$$

In assessing the results, inhibition rates not less than 71%, from 41% to 70%, and not more than 40% were considered to be (+), (±) and (-), respectively, and these are the symbols employed in the following Table.

## Table 4

### Antagonistic effect against reserpine-induced ptosis

| Compound of Example No. | Dose (mg/kg) | Administration route | Maximum inhibition rate (%) | Assessment |
|---|---|---|---|---|
| 1 | 10 | p. o. | 25 | - |
| 1 | 30 | p. o. | 50 | ± |
| 1 | 100 | p. o. | 80 | + |
| 2 | 10 | p. o. | 0 | - |
| 2 | 30 | p. o. | 80 | + |
| 2 | 100 | p. o. | 100 | + |
| 3* | 10 | p. o. | 25 | - |
| 3* | 30 | p. o. | 50 | ± |
| 3* | 100 | p. o. | 100 | + |
| Amitriptyline hydrochloride: | | | | |
| | 10 | p. o. | 67 | ± |
| | 30 | p. o. | 48 | - |
| | 100 | p. o. | 42 | - |
| Imipramine hydrochloride: | | | | |
| | 10 | p. o. | 79 | + |
| | 30 | p. o. | 85 | + |
| | 100 | p. o. | 100 | + |

* The compound of Example 3 was employed as its hydrochloride.

As is shown in Table 4, all of the compounds of the present invention exhibited a dose-dependent anti-reserpine effect at doses of 30 and 100 mg/kg. On the other hand, amitriptyline (which has been clinically used for a long time) showed its strongest inhibition, although only 67%, at a dose of 10 mg/kg, and no dose-dependency could be observed. Imipramine which has been also clinically used, however, showed a dose-dependent anti-reserpine effect. Since the data in Table 4 proved that the compounds of the present invention exhibited an anti-reserpine effect similar to that of amitriptyline or imipramine, both of which have been used as antidepressants for a long time, the compounds of the present invention may be expected to exhibit an antidepressant effect in clinical use.

EXPERIMENT 3

Effect on survival time under hypoxic conditions

The test animals employed were male adult mice (5 weeks old and about 30 g body weight) of the ddy strain. The animals were employed in groups, each containing from 5 to 10 animals. Each animal was administered orally with a test compound in suspension (as in the preceding Experiments). 30 minutes after this administration, 2 or 3 animals were placed in a 1.5 litre gas chamber made of acrylate resin, and a mixture of 4% by volume oxygen and 96% nitrogen was supplied at the rate of 10 litres/minute. The time of death of the animal was measured (determined by the cessation of respiration).

The results are shown in the following Table 5.

Table 5

| Cpd. of Ex. No. | Dose (mg/kg) | Survival time (seconds) | P |
|---|---|---|---|
| Control | - | 114 ± 7 | |
| 1 | 10 | 125 ± 14 | |
| 1 | 30 | 144 ± 25 | |
| 1 | 100 | 303 ± 29 | <0.02 |

P: Significance level (Bonferroni's multiple t-test)

The results were tested for significant differences from the control group (t-test)

As can be seen from the above results, the compounds of the present invention were found to improve significantly the survival time of the test animals under hypoxic conditions.

EXPERIMENT 4

Acute toxicity

Each of the test compounds used in the above Experiments 1 to 3 was suspended in a 0.5% w/v CMC aqueous solution, and 300 mg/kg of the compound was orally administered to each of 3 male adult mice of the ddy strain (weighing 20 to 25 g each) which were then observed for 5 days. At the end of this time, no significant symptoms and no deaths had been observed.

Typically the compounds of the present invention are rapidly absorbed after oral administration, intraduodenal administration or intraperitoneal administration. They are expected to be particularly useful as centrally

acting muscle relaxants for the treatment of cerebral stroke infarction sequelae and of cerebrotraumatic sequelae, and for spasrigido hemiplegia, post-operative sequelae (including tumours of the brain and spinal cord), traumatic sequelae (spinal cord injury, cephalic trauma), amyotrophic lateral sclerosis, cerebral palsy, spinocerebellar degeneration, disorders of the spinal cord vessel, SMON (sub-acute myelo-optic neuropathy), caisson disease, spastic paralysis due to any other spinal cord disease, and increased myotony such as systemic cramp or shoulder stiffness. They are also expected to be useful as cerebro-active drugs for the treatment of the acute and chronic phases of cerebral stroke infarction or for the postoperative treatment of patients with a cerebral tumour, a head injury, or the like.

The compounds of the present invention may be pro-drugs for certain of the compounds of the prior art referred to previously, and have the advantage of showing a rather slow onset of therapeutic activity, as compared with those prior compounds. Moreover, the compounds of the present invention are thought to have much lower toxicities, especially sub-acute and chronic toxicities, than the prior compounds.

The compounds of the present invention may therefore be used in the treatment of such disorders, and, for this purpose, may be formulated as conventional pharmaceutical preparations, as is well known in the art. Thus, the compounds may be administered orally, e.g. in the form of tablets, capsules, granules, powders, syrups, or other such well known forms, or parenterally, e.g. by injections, suppositories, etc.

These pharmaceutical preparations can be prepared by conventional means and may contain known adjuvants of a type commonly used in this field, for example vehicles, binders, disintegrators, lubricants, correctives, etc. depending upon the intended use and form of the preparation. The dose will depend upon the condition, age, and body weight of the patient as well as upon the nature and severity of the disorder to be treated, but in the case of oral administration to an adult human patient, we would normally suggest a total daily dose of from 5 mg to 50 mg, which may be administered in a single dose or in divided doses, e.g. from one to three times a day.

The preparation of the compounds of the present invention is further illustrated by the following Examples, and the formulation of these compounds into pharmaceutically useful dosage forms is illustrated by the subsequent Preparations.

## EXAMPLE 1

### 2-(2-Acetoxy-3-morpholinopropyl)-5-phenyl-3-isoxazolone

A solution of 2.00 g (6.57 mmole) of 2-(2-hydroxy-3-morpholinopropyl)-5-phenyl-3-isoxazolone in 30 ml of dry tetrahydrofuran was cooled at 5°C, and then 1.60 g (15.82 mmole) of triethylamine and 0.62 g (7.88 mmole) of acetyl chloride were added dropwise. The mixture was then stirred for 30 minutes at 3°C to 5°C and then for 1 hour at room temperature. At the end of this time, the reaction solution was poured into 100 ml of water and extracted twice, each time with 100 ml of ethyl acetate. The combined ethyl acetate extracts were dried over anhydrous magnesium sulphate, and then the magnesium sulphate was removed by filtration. The solvent was removed by distillation under reduced pressure, and the resulting solid residue was recrystallized from diisopropyl ether to obtain 1.92 g (84.5 %) of the title compound as colourless and granular crystals melting at 63 - 64°C.

Infrared Absorption Spectrum (KBr), $\nu_{max}$ $cm^{-1}$:
1730 (C=O), 1674 (C=0).

Nuclear Magnetic Resonance Spectrum ($CDC\ell_3$), $\delta$ ppm:
2.06 (3H, singlet);
2.53 (2H twice, each triplet, J = 4.5 Hz);
2.58 (2H, doublet, J = 6.0 Hz);
3.68 (2H twice, each triplet, J = 4.5 Hz);
4.21 (2H, doublet, J = 6.0 Hz);
5.36 (1H, triplet of triplets J = 6.0 Hz);
6.01 (1H, singlet);
7.33 - 7.86 (5H, multiplet).

## EXAMPLE 2

### 2-(2-Methoxycarbonyloxy-3-morpholinopropyl)-5-phenyl-3-isoxazolone

A solution of 2.00 g (6.57 mmole) of 2-(2-hydroxy-3-morpholinopropyl)-5-phenyl-3-isoxazolone in 20 ml of dry tetrahydrofuran was cooled at 5°C, and then 1.60 g (15.82 mmole) of triethylamine and 0.75 g (7.88 mmole)

of methyl chloroformate were added dropwise. The mixture was then stirred for 30 minutes at 3° to 5°C and then for 2 hours at room temperature. At the end of this time, the insoluble matter was removed by filtration. The solvent was removed, from the filtrate by distillation under reduced pressure, and the resulting residue was purified by silica gel column chromatography, using ethyl acetate as the eluent, to obtain 2.03 g (85.2 %) of the title compound as a colourless powder melting at 88 - 89°C.

Infrared Absorption Spectrum (KBr), $\nu_{max}$ $cm^{-1}$:
1743 (C=O), 1669 (C=O).

Nuclear Magnetic Resonance Spectrum ($CDC\ell_3$), δ ppm:
2.55 (2H twice, each triplet, J = 4.5 Hz);
2.63 (2H, doublet, J = 6.0 Hz);
3.68 (2H x 2, each triplet, J = 6.0 Hz);
3.76 (3H, singlet);
4.20 (1H, doublet of doublets, J = 15.0, 6.0 Hz);
4.31 (1H, doublet of doublets, J = 15.0, 3.0 Hz);
5.17 (1H, tripled doublet of doublets, J = 6.0, 6.0, & 3.0 Hz);
6.01 (1H, singlet);
7.36 - 7. 83 (5H, multiplet).

EXAMPLE 3

5-(p-Chlorophenyl)-2-(2-methoxycarboxy-3-morpholinopropyl)-3-isoxazolone

5.84 g (61.8 mmole) of methyl chloroformate were dropped at 5°C into 30 ml of pyridine containing 3.50 g (10.3 mmole) of 5-(p-chlorophenyl)-2-(2-hydroxy-3-morpholinopropyl)-3-isoxazolone, and the mixture was stirred for 2 hours at room temperature. At the end of this time, the reaction solution was condensed by evaporation under reduced pressure, and the residue was poured into 50 ml of a 5% w/v aqueous solution of sodium hydrogencarbonate. The solution was then extracted twice, each time with 50 ml of ethyl acetate. The combined ethyl acetate extracts were dried over anhydrous magnesium sulphate, and then the magnesium sulphate was removed by filtration. The solvent was then removed by distillation under reduced pressure. The residue was purified by column chromatography through silica gel, using a 1 : 1 by volume mixture of ethyl acetate and hexane as the eluent, to obtain 2.23 g (yield 54.6%) of the title compound as a colourless and transparent oil having a refractive index $n_D^{26}$ = 1.5717.

Infrared Absorption Spectrum ($CHC\ell_3$), $\nu_{max}$ $cm^{-1}$:
1760, 1660.

Nuclear Magnetic Resonance Spectrum ($CDC\ell_3$), δ ppm:
2.30 - 2.86 (2H x 3, each multiplet);
3.72 (2H x 2, triplet, J = 4.5 Hz);
3.80 (3H, singlet);
4.10 (2H, doublet, J = 4.5 Hz);
5.54 (1H, broad);
6.06 (1H, singlet);
7.46 (2H, doublet, J = 9.0 Hz);
7.63 (2H, doublet, J = 9.0 Hz).

EXAMPLE 4

5-(p-Chlorophenyl)-2-(2-methoxycarboxy-3-morpholinopropyl)-3-isoxazolone hydrochloride

2.10 g (5.23 mmole) of 5-(p-chlorophenyl)-2-(2-methoxycarboxy-3-morpholinopropyl)-3-isoxazolone were dissolved in 30 ml of ethanol, and 1.6 ml (6.34 mmole) of a 4N solution of hydrogen chloride in dioxane was added dropwise to the resulting solution. The mixture was then stirred for 30 minutes at room temperature, after which the reaction solution was condensed by evaporation under reduced pressure. The solid residue was recrystallized from a 1 : 1 by volume mixture of methanol and ethanol, to obtain 2.25 g (yield 98.1%) of the title compound as colourless needles melting at 159.5° to 160.5°C.

Infrared Absorption Spectrum (KBr), $\nu_{max}$ $cm^{-1}$:
1759, 1676.

Nuclear Magnetic Resonance Spectrum (hexadeuterated dimethyl sulphoxide), δ ppm:

2.90 - 3.70 (2H x 3, each multiplet);
3.70 (3H, singlet);
3.90 (2H x 2, each triplet, J = 4.5 Hz);
4.26 (2H, doublet, J = 1.5 Hz);
5.50 (1H, broad);
6.60 (1H, singlet);
7.68 (2H, doublet, J = 9.0 Hz);
7.86 (2H, doublet; J = 9.0 Hz).

EXAMPLES 5 TO 32

Following a procedure similar to that described in Example 3 or Example 4, the following compounds were synthesized.

EXAMPLE 5

4-Chloro-2-(2-methoxycarboxy-3-morpholinopropyl)-5-phenyl-3-isoxazolone, melting at 94 - 95°C.

EXAMPLE 6

4-Chloro-2-(2-isobutyryloxy-3-morpholinopropyl)-5-phenyl-3-isoxazolone, $n_D^{26}$ = 1.5453.

EXAMPLE 7

4-Chloro-2-(2-isopropoxycarboxy-3-morpholinopropyl)-5-phenyl-3-isoxazolone, $n_D^{26}$ = 1.5388.

EXAMPLE 8

2-(2-Benzoyloxy-3-morpholinopropyl)-4-chloro-5-phenyl-3-isoxazolone, $n_D^{26}$ = 1.5822.

EXAMPLE 9

4-Chloro-2-(3-morpholino-2-phenoxycarboxypropyl)-5-phenyl-3-isoxazolone, melting at 108 - 109°C.

EXAMPLE 10

4-Chloro-2-(3-morpholino-2-phenylacetoxypropyl)-5-phenyl-3-isoxazolone, melting at 90 - 91°C.

EXAMPLE 11

2-(2-Benzyloxycarboxy-3-morpholinopropyl)-4-chloro-5-phenyl-3-isoxazolone, melting at 97 - 98°C.

EXAMPLE 12

4-Chloro-2-[2-methoxycarboxy-3-(1-pyrrolidinyl)propyl]-5-phenyl-3-isoxazolone, $n_D^{26}$ = 1.5552.

EXAMPLE 13

4-Chloro-2-[2-methoxycarboxy-3-(4-methyl-1-piperazinyl)propyl]-5-phenyl-3-isoxazolone, $n_D^{26}$ = 1.6232.

EXAMPLE 14

4-Chloro-2-[2-methoxycarboxy-3-(1-piperidinyl)propyl]-5-phenyl-3-isoxazolone, $n_D^{26}$ = 1.5470

EXAMPLE 15

5-(p-Chlorophenyl)-2-(2-methoxycarboxy-3-morpholinopropyl)-3-isoxazolone, $n_D^{26}$ = 1.5717.

EXAMPLE 16

2-(2-Methoxycarboxy-3-morpholinopropyl)-4-methyl-5-phenyl-3-isoxazolone, $n_D^{26}$ = 1.5416.

EXAMPLE 17

2-(2-Methoxycarboxy-3-morpholinopropyl)-5-methyl-3-isoxazolone, melting at 69 - 70°C.

EXAMPLE 18

5-Chloro-2-(2-methoxycarboxy-3-morpholinopropyl)-2,3-benzoyl-3-isoxazolone, $n_D^{26}$ = 1.5295.

EXAMPLE 19

4-Chloro-2-(2-methoxycarboxy-3-morpholinopropyl)-5-phenyl-3-isoxazolone hydrochloride, melting at 166 - 167°C.

EXAMPLE 20

4-Chloro-2-(2-isopropoxycarboxy-3-morpholinopropyl)-5-phenyl-3-isoxazolone hydrochloride, melting at 183 - 184°C.

EXAMPLE 21

4-Chloro-2-(2-isobutyryloxy-3-morpholinopropyl)-5-phenyl-3-isoxazolone hydrochloride, melting at 162 - 163°C.

EXAMPLE 22

2-(2-Benzoyloxy-3-morpholinopropyl)-4-chloro-5-phenyl-3-isoxazolone hydrochloride, melting at 171 - 172°C.

EXAMPLE 23

4-Chloro-2-(3-morpholino-2-phenoxycarboxypropyl)-5-phenyl-3-isoxazolone hydrochloride, melting at 181.5 - 182. 5°C.

EXAMPLE 24

4-Chloro-2-(3-morpholino-2-phenylacetoxypropyl)-5-phenyl-3-isoxazolone hydrochloride, 181 - 182°C.

EXAMPLE 25

2-(2-Benzyloxycarboxy-3-morpholinopropyl)-4-chloro-5-phenyl-3-isoxazolone hydrochloride, 147 - 148°C.

EXAMPLE 26

4-Chloro-[2-methoxycarboxy-3-(1-pyrrolidinyl)propyl]-5-phenyl-3-isoxazolone hydrochloride, melting at 160 - 161°C.

EXAMPLE 27

4-Chloro-2-[2-methoxycarboxy-3-(4-methyl-1-piperazinyl)propyl]-5-phenyl-3-isoxazolone dihydrochloride, melting at 153 - 154°C.

EXAMPLE 28

4-Chloro-2-[2-methoxycarboxy-3-(1-piperidinyl)propyl]-5-phenyl-3-isoxazolone hydrochloride, melting at 171 - 172°C.

EXAMPLE 29

5-(p-Chlorophenyl)-2-(2-methoxycarboxy-3-morpholinopropyl)-3-isoxazolone hydrochloride, melting at 159.5 - 160.5°C.

EXAMPLE 30

2-(2-Methoxycarboxy-3-morpholinopropyl)-4-methyl-5-phenyl-3-isoxazolone hydrochloride, melting at 167 - 168°C.

EXAMPLE 31

2-(2-Methoxycarboxy-3-morpholinopropyl)-5-methyl-3-isoxazolone hydrochloride, 153 - 154°C.

EXAMPLE 32

5-Chloro-2-(2-methoxycarboxy-3-morpholinopropyl)-1,2-benzoyl-3-isoxazolone hydrochloride, melting at 151 - 152°C.

PREPARATION 1

Capsules:

The following powdery ingredients were mixed:

| | |
|---|---|
| 2-(2-Acetoxy-3-morpholinopropyl)-5-phenyl-3-isoxazolone (Compound prepared in Example 1) | 25.0 mg |
| Lactose | 153.6 mg |
| Corn starch | 100.0 mg |
| Magnesium stearate | 1.4 mg |
| Total | 280.0 mg |

and the resulting mixture was passed through a 60-mesh (Tyler standard) sieve. Capsules were prepared by putting 280 mg of this powder into a No. 3 gelatin capsule.

PREPARATION 2

Tablets:

The following powdery ingredients were mixed:

| | |
|---|---|
| 2-(2-Methoxycarbonyloxy-3-morpholinopropyl)-5-phenyl-3-isoxazolone (Compound prepared in Example 2) | 10.0 mg |
| Corn starch | 25.0 mg |
| Lactose | 83.3 mg |
| HPC (product of Nippon Soda Co., Ltd.) | 1.2 mg |
| Magnesium stearate | 0.5 mg |
| Total | 120 mg |

and 120 mg tablets were prepared by conventional means.

PREPARATION 3

Capsules:

The following powdery ingredients were mixed:

| | |
|---|---|
| 5-(p-Chlorophenyl)-2-(2-methoxycarboxy-3-morpholinopropyl)-3-isoxazolone hydrochloride (the compound prepared in Example 3) | 25.0 mg |
| Lactose | 153.6 mg |
| Corn starch | 100.0 mg |
| Magnesium stearate | 1.4 mg |
| Total | 280.0 mg |

and the resulting mixture was passed through a 60-mesh sieve. 280 mg of the mixture were put into a No. 3 gelatin capsule to prepare the capsules.

PREPARATION 4

Tablets:

The following powdery ingredients were mixed:

| | |
|---|---|
| 5-(p-Chlorophenyl)-2-(2-methoxycarboxy-3-morpholinopropyl)-3-isoxazolone hydrochloride (the compound prepared in Example 3) | 10.0 mg |
| Corn starch | 25.0 mg |
| Lactose | 83.3 mg |
| HPC (product of Nippon Soda Co., Ltd.) | 1.2 mg |
| Magnesium stearate | .5 mg |
| Total | 120.0 mg |

and 120 mg tablets were prepared by conventional means.

## Claims

**Claims for the following Contracting States : AT, BE, CH, DE, DK, FR, GB, IT, LI, LU, NL, SE**

**1.** Compounds of formula (I):

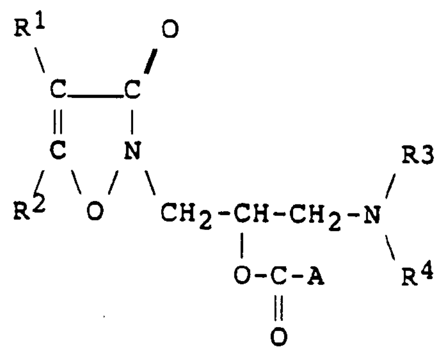

(I)

in which:

$R^1$ represents: a hydrogen atom; a halogen atom; an alkyl group having from 1 to 6 carbon atoms; an alkenyl group having from 2 to 6 carbon atoms; an alkynyl group having from 2 to 6 carbon atoms; an unsubstituted benzyl group; a substituted benzyl group which is substituted by at least one of substituents (a), defined below; an unsubstituted phenyl group; or a substituted phenyl group which is substituted by at least one of substituents (a), defined below;

$R^2$ represents: a hydrogen atom; an alkyl group having from 1 to 6 carbon atoms; an unsubstituted phenyl group; a substituted phenyl group which is substituted by at least one of substituents (a), defined below; or a heterocyclic group having from 5 to 7 ring atoms, of which 1 or 2 are nitrogen and/or oxygen and/or sulphur hetero-atoms, said ring being unsubstituted or being substituted by at least one of substituents (a), defined below, and/or by an oxygen atom; or $R^1$ and $R^2$ together with the carbon atoms to which they are attached, form a hydrocarbon ring having from 5 to 7 ring atoms fused to the isoxazole ring, said hydrocarbon ring being unsubstituted or being substituted by at least one of substituents (a), defined below;

$R^3$ and $R^4$, together with the nitrogen atom to which they are attached, represent an alicyclic amino group having from 5 to 7 ring atoms and having, in addition to the nitrogen atom of the group $-NR^3R^4$, 0 or 1 additional nitrogen and/or oxygen and/or sulphur hetero-atom, said ring being unsubstituted or being substituted by at least one of substituents (a), defined below, and/or by an oxygen atom; and

A represents: an unsubstituted phenyl group; a substituted phenyl group which is substituted by at least one of substituents (a), defined below; an unsubstituted benzyl group; a substituted benzyl group which is substituted by at least one of substituents (a), defined below; an alkyl group having from 1 to 6 carbon atoms; an alkoxy group having from 1 to 6 carbon atoms; an unsubstituted benzyloxy group; a substituted

benzyloxy group which is substituted by at least one of substituents (a), defined below; an unsubstituted phenoxy group; or a substituted phenoxy group which is substituted by at least one of substituents (a), defined below;

substituents (a):

alkyl groups having from 1 to 3 carbon atoms, alkoxy groups having from 1 to 3 carbon atoms, hydroxy groups, halogen atoms, nitro groups, amino groups, aliphatic carboxylic acyl groups having from 2 to 4 carbon atoms and aliphatic carboxylic acylamino groups having from 2 to 4 carbon atoms; and acid addition salts thereof.

**2.** A compound according to Claim 1, in which $R^1$ represents a hydrogen atom, a halogen atom or an alkyl group having from 1 to 4 carbon atoms.

**3.** A compound according to Claim 1 or Claim 2, in which $R^2$ represents a phenyl group or a phenyl group having at least one substituent selected from alkoxy groups having from 1 to 3 carbon atoms, hydroxy groups and halogen atoms.

**4.** A compound according to Claim 1, in which $R^1$ and $R^2$, together with the carbon atoms to which they are attached, represent a 6-membered hydrocarbon ring fused to the isoxazole ring, said hydrocarbon ring being unsubstituted or having at least one halogen substituent.

**5.** A compound according to any one of the preceding Claims, in which $-NR^3R^4$ represents an alicyclic amino group having a total of 5 or 6 ring atoms, of which one is said nitrogen atom and 0 or 1 is an additional nitrogen hetero-atom, said alicyclic amino group being unsubstituted or, where there is an additional nitrogen hetero-atom, having at least one substituent on that additional nitrogen hetero-atom selected from alkyl groups having from 1 to 3 carbon atoms, benzyl groups and phenyl groups.

**6.** A compound according to any one of the preceding Claims, in which A represents an alkyl group having from 1 to 4 carbon atoms, an alkoxy group having from 1 to 4 carbon atoms, a phenyl group, a phenoxy group, a benzyl group or a benzyloxy group, said phenyl, phenoxy, benzyl and benzyloxy groups being unsubstituted or having at least one substituent selected from methoxy groups and halogen atoms.

**7.** A compound according to Claim 1 or Claim 2, in which $R^2$ represents a phenyl group or a phenyl group having at least one substituent selected from the group consisting of methoxy groups and halogen atoms.

**8.** A compound according to Claim 1, in which $R^1$ and $R^2$, together with the carbon atoms to which they are attached, represent a benzene ring fused to the isoxazole ring, said benzene ring being unsubstituted or having one halogen substituent.

**9.** A compound according to any one of Claims 1, 7 and 8, in which $-NR^3R^4$ represents a morpholino group, a piperazinyl group, a methyl-substituted piperazinyl group, a 1-pyrrolidinyl group or a piperidyl group.

**10.** A compound according to Claim 9, in which $-NR^3R^4$ represents a morpholino group, a 1-piperazinyl group, a 4-methyl-1-piperazinyl group, a 1-pyrrolidinyl group or a piperidino group.

**11.** A compound according to any one of Claims 1 and 7 to 10, in which A represents an alkyl group having from 1 to 4 carbon atoms, an alkoxy group having from 1 to 4 carbon atoms, or an unsubstituted phenyl, phenoxy, benzyl or benzyloxy group.

**12.** A compound according to Claim 11, in which A represents a methyl group, a methoxy group, or an unsubstituted phenyl, phenoxy, benzyl or benzyloxy group.

**13.** A compound according to Claim 1, in which:
$R^1$ represents a hydrogen atom, a halogen atom or an alkyl group having from 1 to 4 carbon atoms;
$R^2$ represents a phenyl group or a phenyl group having a halogen substituent;
$-NR^3R^4$ represents a morpholino, piperidyl or piperazinyl group; and
A represents an alkyl or alkoxy group having from 1 to 4 carbon atoms.

**14.** A compound according to Claim 1, in which:
$R^1$ represents a hydrogen atom, a halogen atom or an alkyl group having from 1 to 4 carbon atoms;
$R^2$ represents a phenyl group or a phenyl group having a single halogen substituent;

$-NR^3R^4$ represents a morpholino group; and
A represents an alkoxy group having from 1 to 4 carbon atoms.

**15.** A compound according to Claim 14, in which $R^1$ represents a hydrogen atom, a chlorine atom or a methyl or ethyl group.

**16.** 2-(2-Acetoxy-3-morpholinopropyl)-5-(p-chlorophenyl)-3-isoxazolone and pharmaceutically acceptable salts thereof.

**17.** 5-(p-Chlorophenyl)-2-(2-propionyloxy-3-morpholinopropyl)-3-isoxazolone and pharmaceutically acceptable salts thereof.

**18.** 2-(2-Methoxycarbonyloxy-3-morpholinopropyl)-5-phenyl-3-isoxazolone and pharmaceutically acceptable salts thereof.

**19.** 2-(2-Ethoxycarbonyloxy-3-morpholinopropyl)-5-phenyl-3-isoxazolone and pharmaceutically acceptable salts thereof.

**20.** 4-Chloro-2-(2-methoxycarbonyloxy-3-morpholinopropyl)-5-phenyl-3-isoxazolone and pharmaceutically acceptable salts thereof.

**21.** 4-Chloro-2-(2-ethoxycarbonyloxy-3-morpholinopropyl)-5-phenyl-3-isoxazolone and pharmaceutically acceptable salts thereof.

**22.** 2-(2-Methoxycarbonyloxy-3-morpholinopropyl)-4-methyl-5-phenyl-3-isoxazolone and pharmaceutically acceptable salts thereof.

**23.** 4-Ethyl-2-(2-methoxycarbonyloxy-3-morpholinopropyl)-5-phenyl-3-isoxazolone and pharmaceutically acceptable salts thereof.

**24.** 5-(p-Fluorophenyl)-2-(2-methoxycarboxy-3-morpholinopropyl)-3-isoxazolone and pharmaceutically acceptable salts thereof.

**25.** At least one compound according to any one of Claims 1 to 24 for use in the treatment of cerebrovascular disorders, for use as a centrally-acting muscle relaxant or for use as an antidepressant.

**26.** The use of at least one compound according to any one of Claims 1 to 24 for the manufacture of a medicament for the treatment of cerebrovascular disorders, for use as a centrally- acting muscle relaxant or for use as an antidepressant.

**27.** The use according to Claim 26, in which the depression to be treated is senile depression.

**28.** A pharmaceutical composition for the treatment of cerebrovascular disorders or as a centrally-acting muscle relaxant, which composition comprises an effective amount of an active compound, in which the active compound is a compound according to any one of Claims 1 to 24.

**29.** A process for preparing a compound according to any one of Claims 1 to 24, which process comprises esterifying a compound of formula (II):

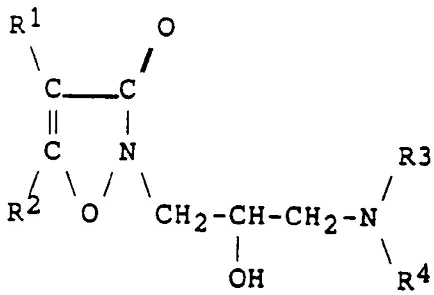

(II)

(in which $R^1$, $R^2$ and $-NR^3R^4$ are as defined in Claim 1), or such a compound in which any active groups other than the hydroxy group to be esterified are protected, with a carboxylic acid of formula (III):

HO-CO-A (III)

(in which A is as defined in Claim 1) or with a reactive derivative thereof.

**30.** A process according to Claim 29, in which the reactive derivative of said carboxylic acid of formula (III) is the halide of formula (IV):

X-CO-A (III)

(in which A is as defined in Claim 1 and X represents a halogen atom).

**Claims for the following Contracting States : ES, GR**

**1.** A process for preparing a compound of formula (I):

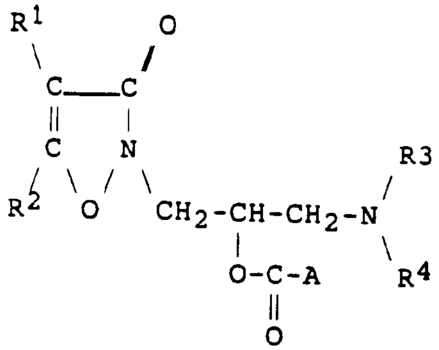

(I)

in which:
$R^1$ represents: a hydrogen atom; a halogen atom; an alkyl group having from 1 to 6 carbon atoms; an alkenyl group having from 2 to 6 carbon atoms; an alkynyl group having from 2 to 6 carbon atoms; an unsubstituted benzyl group; a substituted benzyl group which is substituted by at least one of substituents (a), defined below; an unsubstituted phenyl group; or a substituted phenyl group which is substituted by at least one of substituents (a), defined below;
$R^2$ represents: a hydrogen atom; an alkyl group having from 1 to 6 carbon atoms; an unsubstituted phenyl group; a substituted phenyl group which is substituted by at least one of substituents (a), defined below; or a heterocyclic group having from 5 to 7 ring atoms, of which 1 or 2 are nitrogen and/or oxygen and/or sulphur hetero-atoms, said ring being unsubstituted or being substituted by at least one of substituents (a), defined below, and/or by an oxygen atom; or $R^1$ and $R^2$ together with the carbon atoms to which they are attached, form a hydrocarbon ring having from 5 to 7 ring atoms fused to the isoxazole ring, said hydrocarbon ring being unsubstituted or being substituted by at least one of substituents (a), defined below;
$R^3$ and $R^4$, together with the nitrogen atom to which they are attached, represent an alicyclic amino group having from 5 to 7 ring atoms and having, in addition to the nitrogen atom of the group $-NR^3R^4$, 0 or 1 additional nitrogen and/or oxygen and/or sulphur hetero-atom, said ring being unsubstituted or being substituted by at least one of substituents (a), defined below, and/or by an oxygen atom; and
A represents: an unsubstituted phenyl group; a substituted phenyl group which is substituted by at least one of substituents (a), defined below; an unsubstituted benzyl group; a substituted benzyl group which is substituted by at least one of substituents (a), defined below; an alkyl group having from 1 to 6 carbon atoms; an alkoxy group having from 1 to 6 carbon atoms; an unsubstituted benzyloxy group; a substituted benzyloxy group which is substituted by at least one of substituents (a), defined below; an unsubstituted phenoxy group; or a substituted phenoxy group which is substituted by at least one of substituents (a), defined below;
<u>substituents (a):</u>

alkyl groups having from 1 to 3 carbon atoms, alkoxy groups having from 1 to 3 carbon atoms, hydroxy groups, halogen atoms, nitro groups, amino groups, aliphatic carboxylic acyl groups having from 2 to 4 carbon atoms and aliphatic carboxylic acylamino groups having from 2 to 4 carbon atoms;

or an acid addition salt thereof, which process comprises esterifying a compound of formula (II):

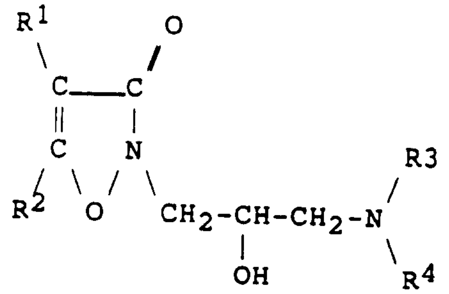

(II)

(in which $R^1$, $R^2$ and $-NR^3R^4$ are as defined above), or such a compound in which any active groups other than the hydroxy group to be esterified are protected, with a carboxylic acid of formula (III):

HO-CO-A (III)

(in which A is as defined above) or with a reactive derivative thereof.

2. A process according to Claim 1, in which the reagents and reaction conditions are so chosen as to prepare a compound of formula (I) or a salt thereof, in which $R^1$ represents a hydrogen atom, a halogen atom or an alkyl group having from 1 to 4 carbon atoms.

3. A process according to Claim 1 or Claim 2, in which the reagents and reaction conditions are so chosen as to prepare a compound of formula (I) or a salt thereof, in which $R^2$ represents a phenyl group or a phenyl group having at least one substituent selected from alkoxy groups having from 1 to 3 carbon atoms, hydroxy groups and halogen atoms.

4. A process according to Claim 1, in which the reagents and reaction conditions are so chosen as to prepare a compound of formula (I) or a salt thereof, in which $R^1$ and $R^2$, together with the carbon atoms to which they are attached, represent a 6-membered hydrocarbon ring fused to the isoxazole ring, said hydrocarbon ring being unsubstituted or having at least one halogen substituent.

5. A process according to any one of the preceding Claims, in which the reagents and reaction conditions are so chosen as to prepare a compound of formula (I) or a salt thereof, in which $-NR^3R^4$ represents an alicyclic amino group having a total of 5 or 6 ring atoms, of which one is said nitrogen atom and 0 or 1 is an additional nitrogen hetero-atom, said alicyclic amino group being unsubstituted or, where there is an additional nitrogen hetero-atom, having at least one substituent on that additional nitrogen hetero-atom selected from alkyl groups having from 1 to 3 carbon atoms, benzyl groups and phenyl groups.

6. A process according to any one of the preceding Claims, in which the reagents and reaction conditions are so chosen as to prepare a compound of formula (I) or a salt thereof, in which A represents an alkyl group having from 1 to 4 carbon atoms, an alkoxy group having from 1 to 4 carbon atoms, a phenyl group, a phenoxy group, a benzyl group or a benzyloxy group, said phenyl, phenoxy, benzyl and benzyloxy groups being unsubstituted or having at least one substituent selected from methoxy groups and halogen atoms.

7. A process according to Claim 1 or Claim 2, in which the reagents and reaction conditions are so chosen as to prepare a compound of formula (I) or a salt thereof, in which $R^2$ represents a phenyl group or a phenyl group having at least one substituent selected from the group consisting of methoxy groups and halogen atoms.

8. A process according to Claim 1, in which the reagents and reaction conditions are so chosen as to prepare a compound of formula (I) or a salt thereof, in which $R^1$ and $R^2$, together with the carbon atoms to which they are attached, represent a benzene ring fused to the isoxazole ring, said benzene ring being unsubstituted or having one halogen substituent.

9. A process according to any one of Claims 1, 7 and 8, in which the reagents and reaction conditions are so chosen as to prepare a compound of formula (I) or a salt thereof, in which $-NR^3R^4$ represents a morpholino group, a piperazinyl group, a methyl-substituted piperazinyl group, a 1-pyrrolidinyl group or a piperidyl group.

**10.** A process according to Claim 9, in which the reagents and reaction conditions are so chosen as to prepare a compound of formula (I) or a salt thereof, in which $-NR^3R^4$ represents a morpholino group, a 1-piperazinyl group, a 4-methyl-1-piperazinyl group, a 1-pyrrolidinyl group or a piperidino group.

**11.** A process according to any one of Claims 1 and 7 to 10, in which the reagents and reaction conditions are so chosen as to prepare a compound of formula (I) or a salt thereof, in which A represents an alkyl group having from 1 to 4 carbon atoms, an alkoxy group having from 1 to 4 carbon atoms, or an unsubstituted phenyl, phenoxy, benzyl or benzyloxy group.

**12.** A process according to Claim 11, in which the reagents and reaction conditions are so chosen as to prepare a compound of formula (I) or a salt thereof, in which A represents a methyl group, a methoxy group, or an unsubstituted phenyl, phenoxy, benzyl or benzyloxy group.

**13.** A process according to Claim 1, in which the reagents and reaction conditions are so chosen as to prepare a compound of formula (I) or a salt thereof, in which:
$R^1$ represents a hydrogen atom, a halogen atom or an alkyl group having from 1 to 4 carbon atoms;
$R^2$ represents a phenyl group or a phenyl group having a halogen substituent;
$-NR^3R^4$ represents a morpholino, piperidyl or piperazinyl group; and
A represents an alkyl or alkoxy group having from 1 to 4 carbon atoms.

**14.** A process according to Claim 1, in which the reagents and reaction conditions are so chosen as to prepare a compound of formula (I) or a salt thereof, in which:
$R^1$ represents a hydrogen atom, a halogen atom or an alkyl group having from 1 to 4 carbon atoms;
$R^2$ represents a phenyl group or a phenyl group having a single halogen substituent;
$-NR^3R^4$ represents a morpholino group; and
A represents an alkoxy group having from 1 to 4 carbon atoms.

**15.** A process according to Claim 14, in which the reagents and reaction conditions are so chosen as to prepare a compound of formula (I) or a salt thereof, in which $R^1$ represents a hydrogen atom, a chlorine atom or a methyl or ethyl group.

**16.** A process according to Claim 1, in which the reagents and reaction conditions are so chosen as to prepare:
2-(2-acetoxy-3-morpholinopropyl)-5-(p-chlorophenyl)-3-isoxazolone;
5-(p-chlorophenyl)-2-(2-propionyloxy-3-morpholinopropyl)-3-isoxazolone;
2-(2-methoxycarbonyloxy-3-morpholinopropyl)-5-phenyl-3-isoxazolone;
2-(2-ethoxycarbonyloxy-3-morpholinopropyl)-5-phenyl-3-isoxazolone;
4-chloro-2-(2-methoxycarbonyloxy-3-morpholinopropyl)-5-phenyl-3-isoxazolone;
4-chloro-2-(2-ethoxycarbonyloxy-3-morpholinopropyl)-5-phenyl-3-isoxazolone;
2-(2-methoxycarbonyloxy-3-morpholinopropyl)-4-methyl-5-phenyl-3-isoxazolone;
4-ethyl-2-(2-methoxycarbonyloxy-3-morpholinopropyl)-5-phenyl-3-isoxazolone;
5-(p-fluorophenyl)-2-(2-methoxycarboxy-3-morpholino-propyl)-3-isoxazolone;
or a pharmaceutically acceptable salt thereof.

**17.** A process according to any one of Claims 1 to 16, in which the reactive derivative of said carboxylic acid of formula (III) is the halide of formula (IV):

$$X\text{-}CO\text{-}A \qquad (III)$$

(in which A is as defined in Claim 1 and X represents a halogen atom).

**18.** A process for preparing a pharmaceutical composition for the treatment of cerebrovascular disorders or as a centrally-acting muscle relaxant, which comprises mixing a compound of formula (I) or a pharmaceutically acceptable acid addition salt thereof, as defined in any one of Claims 1 to 16 with a pharmaceutically acceptable carrier or diluent.

**19.** The use of at least one compound as defined in any one of Claims 1 to 16 for the treatment of cerebrovascular disorders, for use as a centrally-acting muscle relaxant or for use as an antidepressant.

**20.** The use of at least one compound as defined in any one of Claims 1 to 16 for the manufacture of a medicament for the treatment of cerebrovascular disorders, for use as a centrally- acting muscle relaxant or for use as an antidepressant.

## Patentansprüche

### Patentansprüche für folgende Vertragsstaaten : AT, BE, CH, DE, DK, FR, GB, IT, LI, LU, NL, SE

1. Verbindungen der Formel (I)

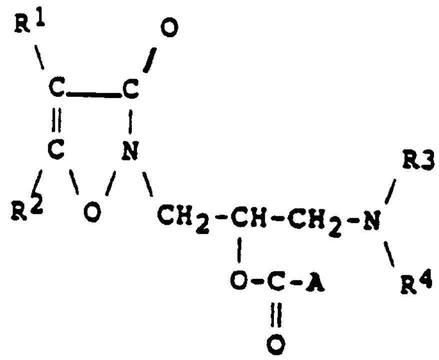

(I)

worin

$R^1$ ein Wasserstoffatom, ein Halogenatom, eine Alkylgruppe mit 1 bis 6 Kohlenstoffatomen, eine Alkenylgruppe mit 2 bis 6 Kohlenstoffatomen, eine Alkinylgruppe mit 2 bis 6 Kohlenstoffatomen, eine unsubstituierte Benzylgruppe, eine substituierte Benzylgruppe, die mit mindestens einem der nachstehend definierten Substituenten (a) substituiert ist, eine unsubstituierte Phenylgruppe oder eine substituierte Phenylgruppe, die mit mindestens einem der nachstehend definierten Substituenten (a) substituiert ist, darstellt,

$R^2$ ein Wasserstoffatom, eine Alkylgruppe mit 1 bis 6 Kohlenstoffatomen, eine unsubstituierte Phenylgruppe, eine substituierte Phenylgruppe, die mit mindestens einem der nachstehend definierten Substituenten (a) substituiert ist, oder eine heterocyclische Gruppe mit 5 bis 7 Ringatomen, von denen 1 oder 2 Stickstoff- und/oder Sauerstoff- und/oder Schwefel-Heteroatome sind, wobei der Ring unsubstituiert ist oder mit mindestens einem der nachstehend definierten Substituenten (a) und/oder mit einem Sauerstoffatom substituiert ist, bedeutet, oder $R^1$ und $R^2$ zusammen mit den Kohlenstoffatomen, an die sie gebunden sind, einen Kohlenwasserstoffring mit 5 bis 7 Ringatomen, der an den Isoxazolring ankondensiert ist, bilden, wobei der Kohlenwasserstoffring unsubstituiert ist oder mit mindestens einem der nachstehend definierten Substituenten (a) substituiert ist,

$R^3$ und $R^4$ zusammen mit dem Stickstoffatom, an das sie gebunden sind, eine alicyclische Aminogruppe mit 5 bis 7 Ringatomen, die zusätzlich zu dem Stickstoffatom der Gruppe $-NR^3R^4$, 0 oder 1 zusätzliches Stickstoff- und/oder Sauerstoff- und/oder Schwefel-Heteroatom aufweist, wobei der Ring unsubstituiert ist oder mit mindestens einem der nachstehend definierten Substituenten (a) und/oder durch ein Sauerstoffatom substituiert ist, bedeuten, und

A eine unsubstituierte Phenylgruppe, eine substituierte Phenylgruppe, die mit mindestens einem der nachstehend definierten Substituenten (a) substituiert ist, eine unsubstituierte Benzylgruppe, eine substituierte Benzylgruppe, die mit mindestens einem der nachstehend definierten Substituenten (a) substituiert ist, eine Alkylgruppe mit 1 bis 6 Kohlenstoffatomen, eine Alkoxygruppe mit 1 bis 6 Kohlenstoffatomen, eine unsubstituierte Benzyloxygruppe, eine substituierte Benzyloxygruppe, die mit mindestens einem der nachstehend definierten Substituenten (a) substituiert ist, eine unsubstituierte Phenoxygruppe oder eine substituierte Phenoxygruppe, die mit mindestens einem der nachstehend definierten Substituenten (a) substituiert ist, bedeutet;

<u>Substituenten (a):</u>

Alkylgruppen mit 1 bis 3 Kohlenstoffatomen, Alkoxygruppen mit 1 bis 3 Kohlenstoffatomen, Hydroxygruppen, Halogenatome, Nitrogruppen, Aminogruppen, Acylgruppen von aliphatischen Carbonsäuren mit 2 bis 4 Kohlenstoffatomen und Acylaminogruppen von aliphatischen Carbonsäuren mit 2 bis 4 Kohlenstoffatomen,

und Säureadditionssalze dieser Verbindungen.

2. Verbindung nach Anspruch 1, in der $R^1$ ein Wasserstoffatom, ein Halogenatom oder eine Alkylgruppe mit 1 bis 4 Kohlenstoffatomen bedeutet.

3. Verbindung nach Anspruch 1 oder Anspruch 2, in der $R^2$ eine Phenylgruppe oder eine Phenylgruppe mit mindestens einem Substituenten bedeutet, der unter Alkoxygruppen mit 1 bis 3 Kohlenstoffatomen, Hydroxygruppen und Halogenatomen ausgewählt ist.

4. Verbindung nach Anspruch 1, in der $R^1$ und $R^2$ gemeinsam mit den Kohlenstoffatomen, an die sie gebunden sind, einen an den Isoxazolring ankondensierten, sechsgliedrigen Kohlenwasserstoffring darstellen, wobei der Kohlenwasserstoffring unsubstituiert ist oder mindestens einen Halogensubstituenten aufweist.

5. Verbindung nach einem der vorhergehenden Ansprüche, worin $-NR^3R^4$ eine alicyclische Aminogruppe mit insgesamt 5 oder 6 Ringatomen bedeutet, von denen eines dieses Stickstoffatom ist und 0 oder 1 ein zusätzliches Stickstoff-Heteroatom ist, wobei die alicyclische Aminogruppe unsubstituiert ist oder, wenn ein zusätzliches Stickstoff-Heteroatom vorliegt, an dem zusätzlichen Stickstoff-Heteroatom mindestens einen Substituenten aufweist, der unter Alkylgruppen mit 1 bis 3 Kohlenstoffatomen, Benzylgruppen und Phenylgruppen ausgewählt ist.

6. Verbindung nach einem der vorhergehenden Ansprüche, worin A eine Alkylgruppe mit 1 bis 4 Kohlenstoffatomen, eine Alkoxygruppe mit 1 bis 4 Kohlenstoffatomen, eine Phenylgruppe, eine Phenoxygruppe, eine Benzylgruppe oder eine Benzyloxygruppe bedeutet, wobei die Phenyl-, Phenoxy-, Benzyl- und Benzyloxygruppe unsubstituiert ist oder mindestens einen unter Methoxygruppen und Halogenatomen ausgewählten Substituenten aufweist.

7. Verbindung nach Anspruch 1 oder Anspruch 2, worin $R^2$ eine Phenylgruppe oder eine Phenylgruppe bedeutet, die mindestens einen unter Methoxygruppen und Halogenatomen ausgewählten Substituenten aufweist.

8. Verbindung nach Anspruch 1, worin $R^1$ und $R^2$ gemeinsam mit den Kohlenstoffatomen, an die sie gebunden sind, einen an den Isoxazolring ankondensierten Benzolring darstellen, wobei der Benzolring unsubstituiert ist oder einen Halogensubstituenten aufweist.

9. Verbindung nach einem der Ansprüche 1, 7 und 8, worin $-NR^3R^4$ eine Morpholinogruppe, Piperazinylgruppe, eine methylsubstituierte Piperazinylgruppe, eine 1-Pyrrolidinylgruppe oder eine Piperidylgruppe bedeutet.

10. Verbindung nach Anspruch 9, worin $-NR^3R^4$ eine Morpholinogruppe, eine 1-Piperazinylgruppe, eine 4-Methyl-1-piperazinylgruppe, eine 1-Pyrrolidinylgruppe oder eine Piperidinogruppe bedeutet.

11. Verbindung nach einem der Ansprüche 1 und 7 bis 10, worin A eine Alkylgruppe mit 1 bis 4 Kohlenstoffatomen, eine Alkoxygruppe mit 1 bis 4 Kohlenstoffatomen oder eine unsubstituierte Phenyl-, Phenoxy-, Benzyl- oder Benzyloxygruppe bedeutet.

12. Verbindung nach Anspruch 11, worin A eine Methylgruppe, eine Methoxygruppe oder eine unsubstituierte Phenyl-, Phenoxy-, Benzyl- oder Benzyloxygruppe bedeutet.

13. Verbindung nach Anspruch 1, worin
$R^1$ ein Wasserstoffatom, ein Halogenatom oder eine Alkylgruppe mit 1 bis 4 Kohlenstoffatomen bedeutet,
$R^2$ eine Phenylgruppe oder eine Phenylgruppe mit einem Halogensubstituenten bedeutet,
$-NR^3R^4$ eine Morpholino-, Piperidyl- oder Piperazinylgruppe bedeutet, und
A eine Alkyl- oder Alkoxygruppe mit 1 bis 4 Kohlenstoffatomen bedeutet.

14. Verbindung nach Anspruch 1, worin
$R^1$ ein Wasserstoffatom, ein Halogenatom oder eine Alkylgruppe mit 1 bis 4 Kohlenstoffatomen bedeutet,
$R^2$ eine Phenylgruppe oder einer Phenylgruppe mit einem einzigen Halogensubstituenten bedeutet,
$-NR^3R^4$ eine Morpholinogruppe bedeutet, und
A eine Alkoxygruppe mit 1 bis 4 Kohlenstoffatomen bedeutet.

15. Verbindung nach Anspruch 14, worin $R^1$ ein Wasserstoffatom, ein Chloratom oder eine Methyl- oder Ethylgruppe bedeutet.

16. 2-(2-Acetoxy-3-morpholinopropyl)-5-(p-chlorphenyl)-3-isoxazolon und pharmazeutisch geeignete Salze

davon.

**17.** 5-(p-Chlorphenyl)-2-(2-propionyloxy-3-morpholinopropyl)-3-isoxazolon und pharmazeutisch geeignete Salze davon.

**18.** 2-(2-Methoxycarbonyloxy-3-morpholinopropyl)-5-phenyl-3-isoxazolon und pharmazeutisch geeignete Salze davon.

**19.** 2-(2-Ethoxycarbonyloxy-3-morpholinopropyl)-5-phenyl-3-isoxazolon und pharmazeutisch geeignete Salze davon.

**20.** 4-Chlor-2-(2-methoxycarbonyloxy-3-morpholinopropyl)-5-phenyl-3-isoxazolon und pharmazeutisch geeignete Salze davon.

**21.** 4-Chlor-2-(2-ethoxycarbonyloxy-3-morpholinopropyl)-5-phenyl-3-isoxazolon und pharmazeutisch geeignete Salze davon.

**22.** 2-(2-Methoxycarbonyloxy-3-morpholinopropyl)-4-methyl-5-phenyl-3-isoxazolon und pharmazeutisch geeignete Salze davon.

**23.** 4-Ethyl-2-(2-methoxycarbonyloxy-3-morpholinopropyl)-5-phenyl-3-isoxazolon und pharmazeutisch geeignete Salze davon.

**24.** 5-(p-Fluorphenyl)-2-(2-methoxycarboxy-3-morpholinopropyl)-3-isoxazolon und pharmazeutisch geeignete Salze davon.

**25.** Mindestens eine Verbindung nach einem der Ansprüche 1 bis 24 zur Verwendung zur Behandlung von zerebrovaskulären Erkrankungen, zur Verwendung als zentral wirkendes Muskelrelaxans oder zur Verwendung als Antidepressivum.

**26.** Verwendung mindestens einer Verbindung nach einem der Ansprüche 1 bis 24 zur Herstellung eines Arzneimittels zur Behandlung von zerebrovaskulären Erkrankungen, zur Verwendung als zentral wirkendes Muskelrelaxans oder zur Verwendung als Antidepressivum.

**27.** Verwendung nach Anspruch 26, wobei die zu behandelnde Depression Altersdepression ist.

**28.** Arzneimittelzusammensetzung zur Behandlung von zerebrovaskulären Erkrankungen oder als zentral wirkendes Relaxans, wobei die Zusammensetzung eine wirksame Menge einer aktiven Verbindung enthält und die aktive Verbindung eine Verbindung nach einem der Ansprüche 1 bis 24 ist.

**29.** Verfahren zur Herstellung einer Verbindung nach einem der Ansprüche 1 bis 24, das darin besteht, daß eine Verbindung der Formel (II)

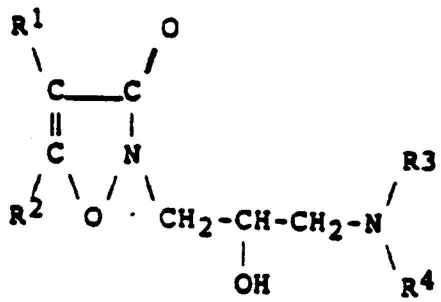

(II)

(worin $R^1$,$R^2$ und $-NR^3R^4$ wie in Anspruch 1 definiert sind) oder eine derartige Verbindung, in der von der zu veresternden Hydroxygruppe verschiedene aktive Gruppen geschützt sind, mit einer Carbonsäure der Formel (III)

**HO-CO-A (III)**

(worin A wie in Anspruch 1 definiert ist) oder mit einem reaktiven Derivat davon verestert wird.

**30.** Verfahren nach Anspruch 29, wobei das reaktive Derivat der Carbonsäure der Formel (III) das Halogenid der Formel (IV) ist

**X-CO-A (IV)**

(wobei A wie in Anspruch 1 definiert ist X ein Halogenatom bedeutet).

**Patentansprüche für folgende Vertragsstaaten : ES, GR**

**1.** Verfahren zur Herstellung einer Verbindung der Formel (I)

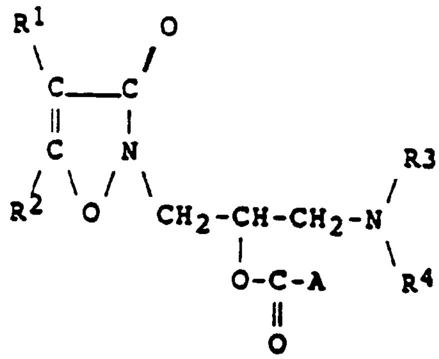

(I)

worin

$R^1$ ein Wasserstoffatom, ein Halogenatom, eine Alkylgruppe mit 1 bis 6 Kohlenstoffatomen, eine Alkenylgruppe mit 2 bis 6 Kohlenstoffatomen, eine Alkinylgruppe mit 2 bis 6 Kohlenstoffatomen, eine unsubstituierte Benzylgruppe, eine substituierte Benzylgruppe, die mit mindestens einem der nachstehend definierten Substituenten (a) substituiert ist, eine unsubstituierte Phenylgruppe oder eine substituierte Phenylgruppe, die mit mindestens einem der nachstehend definierten Substituenten (a) substituiert ist, darstellt,

$R^2$ ein Wasserstoffatom, eine Alkylgruppe mit 1 bis 6 Kohlenstoffatomen, eine unsubstituierte Phenylgruppe, eine substituierte Phenylgruppe, die mit mindestens einem der nachstehend definierten Substituenten (a) substituiert ist, oder eine heterocyclische Gruppe mit 5 bis 7 Ringatomen, von denen 1 oder 2 Stickstoff- und/oder Sauerstoff- und/oder Schwefel-Heteroatome sind, wobei der Ring unsubstituiert ist oder mit mindestens einem der nachstehend definierten Substituenten (a) und/oder mit einem Sauerstoffatom substituiert ist, bedeutet, oder $R^1$ und $R^2$ zusammen mit den Kohlenstoffatomen, an die sie gebunden sind, einen Kohlenwasserstoffring mit 5 bis 7 Ringatomen, der an den Isoxazolring ankondensiert ist, bilden, wobei der Kohlenwasserstoffring unsubstituiert ist oder mit mindestens einem der nachstehend definierten Substituenten (a) substituiert ist,

$R^3$ und $R^4$ zusammen mit dem Stickstoffatom, an das sie gebunden sind, eine alicyclische Aminogruppe mit 5 bis 7 Ringatomen, die zusätzlich zu dem Stickstoffatom der Gruppe $-NR^3R^4$, 0 oder 1 zusätzliches Stickstoff- und/oder Sauerstoff- und/oder Schwefel-Heteroatom aufweist, wobei der Ring unsubstituiert ist oder mit mindestens einem der nachstehend definierten Substituenten (a) und/oder durch ein Sauerstoffatom substituiert ist, bedeuten, und

A eine unsubstituierte Phenylgruppe, eine substituierte Phenylgruppe, die mit mindestens einem der nachstehend definierten Substituenten (a) substituiert ist, eine unsubstituierte Benzylgruppe, eine substituierte Benzylgruppe, die mit mindestens einem der nachstehend definierten Substituenten (a) substituiert ist, eine Alkylgruppe mit 1 bis 6 Kohlenstoffatomen, eine Alkoxygruppe mit 1 bis 6 Kohlenstoffatomen, eine unsubstituierte Benzyloxygruppe, eine substituierte Benzyloxygruppe, die mit mindestens einem der nachstehend definierten Substituenten (a) substituiert ist, eine unsubstituierte Phenoxygruppe oder eine substituierte Phenoxygruppe, die mit mindestens einem der nachstehend definierten Substituenten (a) substituiert ist, bedeutet;

<u>Substituenten (a):</u>

Alkylgruppen mit 1 bis 3 Kohlenstoffatomen, Alkoxygruppen mit 1 bis 3 Kohlenstoffatomen, Hydroxygruppen, Halogenatome, Nitrogruppen, Aminogruppen, Acylgruppen von aliphatischen Carbonsäuren mit 2 bis 4 Kohlenstoffatomen und Acylaminogruppen von aliphatischen Carbonsäuren mit 2 bis 4 Kohlenstoffatomen,

oder eines Säureadditionssalzes davon, das darin besteht, daß eine Verbindung der Formel (II)

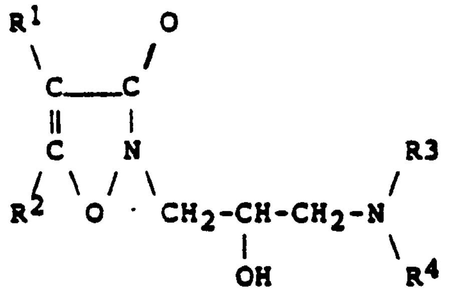

(II)

(worin $R^1$,$R^2$ und $-NR^3R^4$ wie oben definiert sind) oder eine derartige Verbindung, in der von der zu veresternden Hydroxygruppe verschiedene aktive Gruppen geschützt sind, mit einer Carbonsäure der Formel (III)

**HO-CO-A (III)**

(worin A wie oben definiert ist) oder mit einem reaktiven Derivat davon verestert wird.

2. Verfahren nach Anspruch 1, bei dem die Reaktanten und die Reaktionsbedingungen so gewählt werden, daß eine Verbindung der Formel (I) oder eine Salz davon hergestellt wird, worin $R^1$ ein Wasserstoffatom, ein Halogenatom oder eine Alkylgruppe mit 1 bis 4 Kohlenstoffatomen bedeutet.

3. Verfahren nach Anspruch 1 oder Anspruch 2, bei dem die Reaktanten und die Reaktionsbedingungen so gewählt werden, daß eine Verbindung der Formel (I) oder ein Salz davon hergestellt wird, worin $R^2$ eine Phenylgruppe oder eine Phenylgruppe mit mindestens einem Substituenten bedeutet, der unter Alkoxygruppen mit 1 bis 3 Kohlenstoffatomen, Hydroxygruppen und Halogenatomen ausgewählt ist.

4. Verfahren nach Anspruch 1, bei dem die Reaktanten und die Reaktionsbedingungen so gewählt werden, daß eine Verbindung der Formel (I) oder ein Salz davon hergestellt wird, worin $R^1$ und $R^2$ gemeinsam mit den Kohlenstoffatomen, an die sie gebunden sind, einen an den Isoxazolring ankondensierten, sechsgliedrigen Kohlenwasserstoffring darstellen, wobei der Kohlenwasserstoffring unsubstituiert ist oder mindestens einen Halogensubstituenten aufweist.

5. Verfahren nach einem der vorhergehenden Ansprüche, bei dem die Reaktanten und die Reaktionsbedingungen so gewählt werden, daß eine Verbindung der Formel (I) oder ein Salz davon hergestellt wird, worin $-NR^3R^4$ eine alicyclische Aminogruppe mit insgesamt 5 oder 6 Ringatomen bedeutet, von denen eines dieses Stickstoffatom ist und 0 oder 1 ein zusätzliches Stickstoff-Heteroatom ist, wobei die alicyclische Aminogruppe unsubstituiert ist oder, wenn ein zusätzliches Stickstoff-Heteroatom vorliegt, an dem zusätzlichen Stickstoff-Heteroatom mindestens einen Substituenten aufweist, der unter Alkylgruppen mit 1 bis 3 Kohlenstoffatomen, Benzylgruppen und Phenylgruppen ausgewählt ist.

6. Verfahren nach einem der vorhergehenden Ansprüche, bei dem die Reaktanten und die Reaktionsbedingungen so gewählt werden, daß eine Verbindung der Formel (I) oder ein Salz davon hergestellt wird, worin A eine Alkylgruppe mit 1 bis 4 Kohlenstoffatomen, eine Alkoxygruppe mit 1 bis 4 Kohlenstoffatomen, eine Phenylgruppe, eine Phenoxygruppe, eine Benzylgruppe oder eine Benzyloxygruppe bedeutet, wobei die Phenyl-, Phenoxy-, Benzyl- und Benzyloxygruppe unsubstituiert ist oder mindestens einen unter Methoxygruppen und Halogenatomen ausgewählten Substituenten aufweist.

7. Verfahren nach Anspruch 1 oder Anspruch 2, bei dem die Reaktanten und die Reaktionsbedingungen so gewählt werden, daß eine Verbindung der Formel (I) oder ein Salz davon hergestellt wird, worin $R^2$ eine Phenylgruppe oder eine Phenylgruppe bedeutet, die mindestens einen unter Methoxygruppen und Halogenatomen ausgewählten Substituenten aufweist.

8. Verfahren nach Anspruch 1, bei dem die Reaktanten und die Reaktionsbedingungen so gewählt werden, daß eine Verbindung der Formel (I) oder ein Salz davon hergestellt wird, worin $R^1$ und $R^2$ gemeinsam mit den Kohlenstoffatomen, an die sie gebunden sind, einen an den Isoxazolring ankondensierten Benzolring darstellen, wobei der Benzolring unsubstituiert ist oder einen Halogensubstituenten aufweist.

9. Verfahren nach einem der Ansprüche 1, 7 und 8, bei dem die Reaktanten und die Reaktionsbedingungen so gewählt werden, daß eine Verbindung der Formel (I) oder ein Salz davon hergestellt wird, worin $-NR^3R^4$

eine Morpholinogruppe, Piperazinylgruppe, eine methylsubstituierte Piperazinylgruppe, eine 1-Pyrrolidinylgruppe oder eine Piperidylgruppe bedeutet.

**10.** Verfahren nach Anspruch 9, bei dem die Reaktanten und die Reaktionsbedingungen so gewählt werden, daß eine Verbindung der Formel (I) oder ein Salz davon hergestellt wird, worin $-NR^3R^4$ eine Morpholinogruppe, eine 1-Piperazinylgruppe, eine 4-Methyl-1-piperazinylgruppe, eine 1-Pyrrolidinylgruppe oder eine Piperidinogruppe bedeutet.

**11.** Verfahren nach einem der Ansprüche 1 und 7 bis 10, bei dem die Reaktanten und die Reaktionsbedingungen so gewählt werden, daß eine Verbindung der Formel (I) oder ein Salz davon hergestellt wird, worin A eine Alkylgruppe mit 1 bis 4 Kohlenstoffatomen, eine Alkoxygruppe mit 1 bis 4 Kohlenstoffatomen oder eine unsubstituierte Phenyl-, Phenoxy-, Benzyl- oder Benzyloxygruppe bedeutet.

**12.** Verfahren nach Anspruch 11, bei dem die Reaktanten und die Reaktionsbedingungen so gewählt werden, daß eine Verbindung der Formel (I) oder ein Salz davon hergestellt wird, worin A eine Methylgruppe, eine Methoxygruppe oder eine unsubstituierte Phenyl-, Phenoxy-, Benzyl- oder Benzyloxygruppe bedeutet.

**13.** Verfahren nach Anspruch 1, bei dem die Reaktanten und die Reaktionsbedingungen so gewählt werden, daß eine Verbindung der Formel (I) oder ein Salz davon hergestellt wird, worin
$R^1$ ein Wasserstoffatom, ein Halogenatom oder eine Alkylgruppe mit 1 bis 4 Kohlenstoffatomen bedeutet,
$R^2$ eine Phenylgruppe oder eine Phenylgruppe mit einem Halogensubstituenten bedeutet,
$-NR^3R^4$ eine Morpholino-, Piperidyl- oder Piperazinylgruppe bedeutet, und
A eine Alkyl- oder Alkoxygruppe mit 1 bis 4 Kohlenstoffatomen bedeutet.

**14.** Verfahren nach Anspruch 1, bei dem die Reaktanten und die Reaktionsbedingungen so gewählt werden, daß eine Verbindung der Formel (I) oder ein Salz davon hergestellt wird, worin
$R^1$ ein Wasserstoffatom, ein Halogenatom oder eine Alkylgruppe mit 1 bis 4 Kohlenstoffatomen bedeutet,
$R^2$ eine Phenylgruppe oder einer Phenylgruppe mit einem einzigen Halogensubstituenten bedeutet,
$-NR^3R^4$ eine Morpholinogruppe bedeutet, und
A eine Alkoxygruppe mit 1 bis 4 Kohlenstoffatomen bedeutet.

**15.** Verfahren nach Anspruch 14, bei dem die Reaktanten und die Reaktionsbedingungen so gewählt werden, daß eine Verbindung der Formel (I) oder ein Salz davon hergestellt wird, worin $R^1$ ein Wasserstoffatom, ein Chloratom oder eine Methyl- oder Ethylgruppe bedeutet.

**16.** Verfahren nach Anspruch 1, bei dem die Reaktanten und die Reaktionsbedingungen so gewählt werden, daß
2-(2-Acetoxy-3-morpholinopropyl)-5-(p-chlorphenyl)-3-isoxazolon,
5-(p-Chlorphenyl)-2-(2-propionyloxy-3-morpholinopropyl)-3-isoxazolon,
2-(2-Methoxycarbonyloxy-3-morpholinopropyl)-5-phenyl-3-isoxazolon,
2-(2-Ethoxycarbonyloxy-3-morpholinopropyl)-5-phenyl-3-isoxazolon,
4-Chlor-2-(2-methoxycarbonyloxy-3-morpholinopropyl)-5-phenyl-3-isoxazolon,
4-Chlor-2-(2-ethoxycarbonyloxy-3-morpholinopropyl)-5-phenyl-3-isoxazolon,
2-(2-Methoxycarbonyloxy-3-morpholinopropyl)-4-methyl-5-phenyl-3-isoxazolon,
4-Ethyl-2-(2-methoxycarbonyloxy-3-morpholinopropyl)-5-phenyl-3-isoxazolon,
5-(p-Fluorphenyl)-2-(2-methoxycarboxy-3-morpholinopropyl)-3-isoxazolon
oder ein Salz davon hergestellt wird.

**17.** Verfahren nach einem der Ansprüche 1 bis 16, bei dem das reaktive Derivat der Carbonsäure der Formel (III) das Halogenid der Formel (IV) ist

$$X\text{-}CO\text{-}A \qquad (IV)$$

worin A wie in Anspruch 1 definiert ist und X ein Halogenatom bedeutet.

**18.** Verfahren zur Herstellung einer Arzneimittelzusammensetzung zur Behandlung von zerebrovaskulären Erkrankungen oder als zentral wirkendes Muskelrelaxans, das darin besteht, daß eine Verbindung der Formel (I) oder ein pharmazeutisch geeignetes Säureadditionssalz davon gemäß der Definition in einem der Ansprüche 1 bis 16 mit einem pharmazeutisch geeigneten Trägermaterial oder Verdünnungsmittel vermischt wird.

19. Verwendung mindestens einer Verbindung gemäß der Definition in einem der Ansprüche 1 bis 16 zur Behandlung von zerebrovaskulären Erkrankungen, zur Verwendung als zentral wirkendes Muskelrelaxans oder zur Verwendung als Antidepressivum.

20. Verwendung mindestens einer Verbindung gemäß der Definition in einem der Ansprüche 1 bis 16 zur Herstellung eines Arzneimittels zur Behandlung von zerebrovaskulären Erkrankungen, zur Verwendung als zentral wirkendes Muskelrelaxans oder zur Verwendung als Antidepressivum.

## Revendications

**Revendications pour les Etats contractants suivants : AT, BE, CH, DE, DK, FR, GB, IT, LI, LU, NL, SE**

1. Composés de formule (I) :

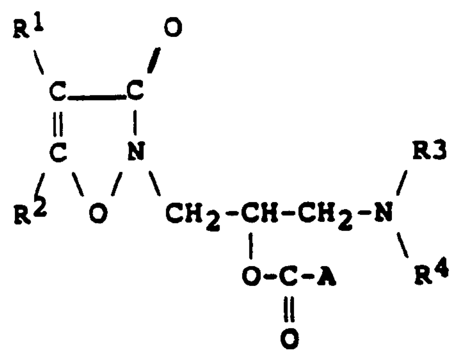

(I)

dans laquelle :

$R^1$ représente : un atome d'hydrogène ; un atome d'halogène ; un groupe alkyle ayant de 1 à 6 atomes de carbone ; un groupe alcényle ayant de 2 à 6 atomes de carbone ; un groupe alcynyle ayant de 2 à 6 atomes de carbone ; un groupe benzyle non substitué ; un groupe benzyle substitué qui est substitué par au moins un des substituants (a) définis ci-après ; un groupe phényle non substitué ; ou un groupe phényle substitué qui est substitué par au moins un des substituants (a) définis ci-après ;

$R^2$ représente : un atome d'hydrogène ; un groupe alkyle ayant de 1 à 6 atomes de carbone ; un groupe phényle non substitué ; un groupe phényle substitué qui est substitué par au moins un des substituants (a) définis ci-après ; ou un groupe hétérocyclique ayant de 5 à 7 atomes formant le cycle, dont 1 ou 2 sont des hétéroatomes d'azote et/ou d'oxygène et/ou de soufre, ledit cycle étant non substitué ou étant substitué par au moins un des substituants (a) définis ci-après et/ou par un atome d'oxygène ; ou $R^1$ et $R^2$, avec les atomes de carbone auxquels ils sont fixés, forment un cycle hydrocarboné ayant de 5 à 7 atomes de carbone condensé au cycle isoxazole, ledit cycle hydrocarboné étant non substitué ou étant substitué par au moins un des substituants (a) définis ci-après ;

$R^3$ et $R^4$, avec l'atome d'azote auquel ils sont fixés, représentent un groupe amino alicyclique ayant de 5 à 7 atomes formant le cycle et ayant, en plus de l'atome d'azote du groupe $-NR^3R^4$, 0 ou 1 hétéroatome additionnel d'azote et/ou d'oxygène et/ou de soufre, ledit cycle étant non substitué ou étant substitué par au moins un des substituants (a) définis ci-après et/ou par un atome d'oxygène ; et

A représente : un groupe phényle non substitué ; un groupe phényle substitué qui est substitué par au moins un des substituants (a) définis ci-après ; un groupe benzyle non substitué ; un groupe benzyle substitué qui est substitué par au moins un des substituants (a) définis ci-après ; un groupe alkyle ayant de 1 à 6 atomes de carbone ; un groupe alcoxy ayant de 1 à 6 atomes de carbone ; un groupe benzyloxy non substitué ; un groupe benzyloxy substitué qui est substitué par au moins un des substituants (a) définis ci-après ; un groupe phénoxy non substitué ; ou un groupe phénoxy substitué qui est substitué par au moins un des substituants (a) définis ci-après ;

<u>substituants (a)</u> :

groupes alkyle ayant de 1 à 3 atomes de carbone, groupes alcoxy ayant de 1 à 3 atomes de carbone, groupes hydroxy, atomes d'halogène, groupes nitro, groupes amino, groupes acyle carboxyliques aliphatiques ayant de 2 à 4 atomes de carbone et groupes acylamino carboxyliques aliphatiques ayant

de 2 à 4 atomes de carbone ;
et leurs sels d'addition d'acides.

2. Composé selon la revendication 1, où $R^1$ représente un atome d'hydrogène, un atome d'halogène ou un groupe alkyle ayant de 1 à 4 atomes de carbone.

3. Composé selon la revendication 1 ou la revendication 2, où $R^2$ représente un groupe phényle ou un groupe phényle ayant au moins un substituant choisi parmi les groupes alcoxy ayant de 1 à 3 atomes de carbone, les groupes hydroxy et les atomes d'halogène.

4. Composé selon la revendication 1, où $R^1$ et $R^2$, avec les atomes de carbone auxquels ils sont fixés, représentent un cycle hydrocarboné à 6 chaînons condensé au cycle isoxazole, ledit cycle hydrocarboné étant non substitué ou ayant au moins un substituant halogène.

5. Composé selon l'une quelconque des revendications précédentes, où $-NR^3R^4$ représente un groupe amino alicyclique ayant au total 5 ou 6 atomes formant le cycle, dont un est ledit atome d'azote et 0 ou 1 est un hétéroatome additionnel d'azote, ledit groupe amino alicyclique étant non substitué, ou, lorsqu'il y a un hétéroatome additionnel d'azote, ayant sur cet hétéroatome d'azote additionnel au moins un substituant choisi parmi les groupes alkyle ayant de 1 à 3 atomes de carbone, les groupes benzyle et les groupes phényle.

6. Composé selon l'une quelconque des revendications précédentes, où A représente un groupe alkyle ayant de 1 à 4 atomes de carbone, un groupe alcoxy ayant de 1 à 4 atomes de carbone, un groupe phényle, un groupe phénoxy, un groupe benzyle ou un groupe benzyloxy, lesdits groupes phényle, phénoxy, benzyle et benzyloxy étant non substitués ou ayant au moins un substituant choisi parmi les groupes méthoxy et les atomes d'halogène.

7. Composé selon la revendication 1 ou la revendication 2, où $R^2$ représente un groupe phényle ou un groupe phényle ayant au moins un substituant choisi parmi les groupes méthoxy et les atomes d'halogène.

8. Composé selon la revendication 1, où $R^1$ et $R^2$, avec les atomes de carbone auxquels ils sont fixés, représentent un cycle benzène condensé au cycle isoxazole, ledit cycle benzène étant non substitué ou ayant un substituant halogène.

9. Composé selon l'une quelconque des revendications 1, 7 et 8, où $-NR^3R^4$ représente un groupe morpholino, un groupe pipérazinyle, un groupe pipérazinyle méthyl-substitué, un groupe 1-pyrrolidinyle ou un groupe pipéridyle.

10. Composé selon la revendication 9, où $-NR^3R^4$ représente un groupe morpholino, un groupe 1-pipérazinyle, un groupe 4-méthyl-1-pipérazinyle, un groupe 1-pyrrolidinyle ou un groupe pipéridino.

11. Composé selon l'une quelconque des revendications 1 et 7 à 10, où A représente un groupe alkyle ayant de 1 à 4 atomes de carbone, un groupe alcoxy ayant de 1 à 4 atomes de carbone ou un groupe phényle, phénoxy, benzyle ou benzyloxy non substitués.

12. Composé selon la revendication 11, où A représente un groupe méthyle, un groupe méthoxy ou un groupe phényle, phénoxy, benzyle ou benzyloxy non substitués.

13. Composé selon la revendication 1, où :
$R^1$ représente un atome d'hydrogène, un atome d'halogène ou un groupe alkyle ayant de 1 à 4 atomes de carbone ;
$R^2$ représente un groupe phényle ou un groupe phényle ayant un substituant halogène ;
$-NR^3R^4$ représente un groupe morpholino, pipéridyle ou pipérazinyle ; et
A représente un groupe alkyle ou alcoxy ayant de 1 à 4 atomes de carbone.

14. Composé selon la revendication 1, où :
$R^1$ représente un atome d'hydrogène, un atome d'halogène ou un groupe alkyle ayant de 1 à 4 atomes de carbone ;
$R^2$ représente un groupe phényle ou un groupe phényle ayant un seul substituant halogène ;
$-NR^3R^4$ représente un groupe morpholino ; et

A représente un groupe alcoxy ayant de 1 à 4 atomes de carbone.

**15.** Composé selon la revendication 14, où $R^1$ représente un atome d'hydrogène, un atome de chlore ou un groupe méthyle ou éthyle.

**16.** 2-(2-acétoxy-3-morpholinopropyl)-5-(p-chlorophényl)-3-isoxazolone et ses sels pharmaceutiquement acceptables.

**17.** 5-(p-chlorophényl)-2-(2-propionyloxy-3-morpholinopropyl)-3-isoxazolone et ses sels pharmaceutiquement acceptables.

**18.** 2-(2-méthoxycarbonyloxy-3-morpholinopropyl)-5-phényl-3-isoxazolone et ses sels pharmaceutiquement acceptables.

**19.** 2-(2-éthoxycarbonyloxy-3-morpholinopropyl)-5-phényl-3-isoxazolone et ses sels pharmaceutiquement acceptables.

**20.** 4-chloro-2-(2-méthoxycarbonyloxy-3-morpholinopropyl)-5-phényl-3-isoxazolone et ses sels pharmaceutiquement acceptables.

**21.** 4-chloro-2-(2-éthoxycarbonyloxy-3-morpholinopropyl)-5- phényl-3-isoxazolone et ses sels pharmaceutiquement acceptables.

**22.** 2-(2-méthoxycarbonyloxy-3-morpholinopropyl)-4-méthyl-5-phényl-3-isoxazolone et ses sels pharmaceutiquement acceptables.

**23.** 4-éthyl-2-(2-méthoxycarbonyloxy-3-morpholinopropyl)-5-phényl-3-isoxazolone et ses sels pharmaceutiquement acceptables.

**24.** 5-(p-fluorophényl)-2-(2-méthoxycarboxy-3-morpholinopropyl)-3-isoxazolone et ses sels pharmaceutiquement acceptables.

**25.** Au moins un composé selon l'une quelconque des revendications 1 à 24 pour l'utilisation dans le traitement des troubles cérébro-vasculaires, pour l'utilisation comme myorelaxant à action centrale ou pour l'utilisation comme antidépresseur.

**26.** Utilisation d'au moins un composé selon l'une quelconque des revendications 1 à 24 pour la préparation d'un médicament pour le traitement des troubles cérébro-vasculaires, pour l'utilisation comme myorelaxant à action centrale ou pour l'utilisation comme antidépresseur.

**27.** Utilisation selon la revendication 26, dans laquelle la dépression à traiter est la dépression sénile.

**28.** Composition pharmaceutique pour le traitement des troubles cérébro-vasculaires ou comme myorelaxant à action centrale, laquelle composition comprend une quantité efficace d'un composé actif, où le composé actif est un composé selon l'une quelconque des revendications 1 à 24.

**29.** Procédé pour préparer un composé selon l'une quelconque des revendications 1 à 24, lequel procédé comprend l'estérification d'un composé de formule (II) :

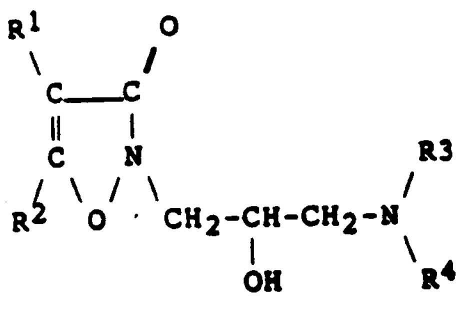

(II)

(dans laquelle $R^1$, $R^2$ et $-NR^3R^4$ sont tels que définis dans la revendication 1), ou d'un tel composé dans lequel tous les groupes actifs autres que le groupe hydroxy à estérifier sont protégés, avec un acide carboxylique de formule (III) :

HO-CO-A (III)

(dans laquelle A est tel que défini dans la revendication 1) ou avec un dérivé réactif de celui-ci.

**30.** Procédé selon la revendication 29, dans lequel le dérivé réactif dudit acide carboxylique de formule (III) est l'halogénure de formule (IV) :

X-CO-A (IV)

(dans laquelle A est tel que défini dans la revendication 1 et X représente un atome d'halogène).

**Revendications pour les Etats contractants suivants : ES, GR**

**1.** Procédé pour préparer un composé de formule (I) :

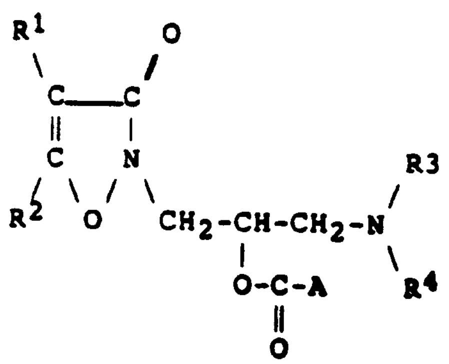

(I)

dans laquelle :

$R^1$ représente : un atome d'hydrogène ; un atome d'halogène ; un groupe alkyle ayant de 1 à 6 atomes de carbone ; un groupe alcényle ayant de 2 à 6 atomes de carbone ; un groupe alcynyle ayant de 2 à 6 atomes de carbone ; un groupe benzyle non substitué ; un groupe benzyle substitué qui est substitué par au moins un des substituants (a) définis ci-après ; un groupe phényle non substitué ; ou un groupe phényle substitué qui est substitué par au moins un des substituants (a) définis ci-après ;

$R^2$ représente : un atome d'hydrogène ; un groupe alkyle ayant de 1 à 6 atomes de carbone ; un groupe phényle non substitué ; un groupe phényle substitué qui est substitué par au moins un des substituants (a) définis ci-après ; ou un groupe hétérocyclique ayant de 5 à 7 atomes formant le cycle, dont 1 ou 2 sont des hétéroatomes d'azote et/ou d'oxygène et/ou de soufre, ledit cycle étant non substitué ou étant substitué par au moins un des substituants (a) définis ci-après et/ou par un atome d'oxygène ; ou $R^1$ et $R^2$, avec les atomes de carbone auxquels ils sont fixés, forment un cycle hydrocarboné ayant de 5 à 7 atomes de carbone condensé au cycle isoxazole, ledit cycle hydrocarboné étant non substitué ou étant substitué par au moins un des substituants (a) définis ci-après ;

$R^3$ et $R^4$, avec l'atome d'azote auquel ils sont fixés, représentent un groupe amino alicyclique ayant de 5 à 7 atomes formant le cycle et ayant, en plus de l'atome d'azote du groupe $-NR^3R^4$, 0 ou 1 hétéroatome additionnel d'azote et/ou d'oxygène et/ou de soufre, ledit cycle étant non substitué ou étant substitué par au moins un des substituants (a) définis ci-après et/ou par un atome d'oxygène ; et

A représente : un groupe phényle non substitué ; un groupe phényle substitué qui est substitué par au moins un des substituants (a) définis ci-après ; un groupe benzyle non substitué ; un groupe benzyle substitué qui est substitué par au moins un des substituants (a) définis ci-après ; un groupe alkyle ayant de 1 à 6 atomes de carbone ; un groupe alcoxy ayant de 1 à 6 atomes de carbone ; un groupe benzyloxy non substitué ; un groupe benzyloxy substitué qui est substitué par au moins un des substituants (a) définis ci-après ; un groupe phénoxy non substitué ; ou un groupe phénoxy substitué qui est substitué par au moins un des substituants (a) définis ci-après ;

<u>substituants (a)</u> :

groupes alkyle ayant de 1 à 3 atomes de carbone, groupes alcoxy ayant de 1 à 3 atomes de carbone, groupes hydroxy, atomes d'halogène, groupes nitro, groupes amino, groupes acyle carboxyliques aliphatiques ayant de 2 à 4 atomes de carbone et groupes acylamino carboxyliques aliphatiques ayant de 2 à 4 atomes de carbone ;

ou un sel d'addition d'acide de celui-ci, lequel procédé comprend l'estérification d'un composé de formule (II) :

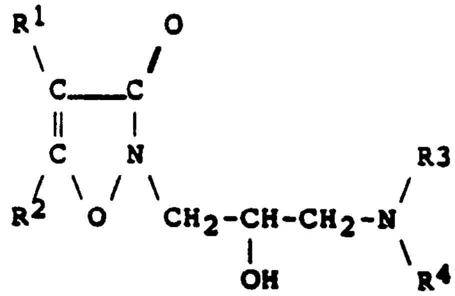

(II)

(dans laquelle $R^1$, $R^2$ et $-NR^3R^4$ sont tels que définis ci-dessus, ou d'un tel composé dans lequel tous les groupes actifs autres que le groupe hydroxy à estérifier sont protégés, avec un acide carboxylique de formule (III) :

HO-CO-A (III)

(dans laquelle A est tel que défini ci-dessus) ou avec un dérivé réactif de celui-ci.

2. Procédé selon la revendication 1, dans lequel les composés réagissants et les conditions de réaction sont choisis pour préparer un composé de formule (I) ou un sel de celui-ci où $R^1$ représente un atome d'hydrogène, un atome d'halogène ou un groupe alkyle ayant de 1 à 4 atomes de carbone.

3. Procédé selon la revendication 1 ou la revendication 2, dans lequel les composés réagissants et les conditions de réaction sont choisis pour préparer un composé de formule (I) ou un sel de celui-ci où $R^2$ représente un groupe phényle ou un groupe phényle ayant au moins un substituant choisi parmi les groupes alcoxy ayant de 1 à 3 atomes de carbone, les groupes hydroxy et les atomes d'halogène.

4. Procédé selon la revendication 1, dans lequel les composés réagissants et les conditions de réaction sont choisis pour préparer un composé de formule (I) ou un sel de celui-ci où $R^1$ et $R^2$, avec les atomes de carbone auxquels ils sont fixés, représentent un cycle hydrocarboné à 6 chaînons condensé au cycle isoxazole, ledit cycle hydrocarboné étant non substitué ou ayant au moins un substituant halogène.

5. Procédé selon l'une quelconque des revendications précédentes, dans lequel les composés réagissants et les conditions de réaction sont choisis pour préparer un composé de formule (I) ou un sel de celui-ci où $-NR^3R^4$ représente un groupe amino alicyclique ayant au total 5 ou 6 atomes formant le cycle, dont un est ledit atome d'azote et 0 ou 1 est un hétéroatome additionnel d'azote, ledit groupe amino alicyclique étant non substitué, ou, lorsqu'il y a un hétéroatome additionnel d'azote, ayant sur cet hétéroatome d'azote additionnel au moins un substituant choisi parmi les groupes alkyle ayant de 1 à 3 atomes de carbone, les groupes benzyle et les groupes phényle.

6. Procédé selon l'une quelconque des revendications précédentes, dans lequel les composés réagissants et les conditions de réaction sont choisis pour préparer un composé de formule (I) ou un sel de celui-ci où A représente un groupe alkyle ayant de 1 à 4 atomes de carbone, un groupe alcoxy ayant de 1 à 4 atomes de carbone, un groupe phényle, un groupe phénoxy, un groupe benzyle ou un groupe benzyloxy, lesdits groupes phényle, phénoxy, benzyle et benzyloxy étant non substitués ou ayant au moins un substituant choisi parmi les groupes méthoxy et les atomes d'halogène.

7. Procédé selon la revendication 1 ou la revendication 2, dans lequel les composés réagissants et les conditions de réaction sont choisis pour préparer un composé de formule (I) ou un sel de celui-ci où $R^2$ représente un groupe phényle ou un groupe phényle ayant au moins un substituant choisi parmi les groupes méthoxy et les atomes d'halogène.

8. Procédé selon la revendication 1, dans lequel les composés réagissants et les conditions de réaction sont choisis pour préparer un composé de formule (I) ou un sel de celui-ci où $R^1$ et $R^2$, avec les atomes de carbone auxquels ils sont fixés, représentent un cycle benzène condensé au cycle isoxazole, ledit cycle benzène étant non substitué ou ayant un substituant halogène.

9. Procédé selon l'une quelconque des revendications 1, 7 et 8, dans lequel les composés réagissants et les conditions de réaction sont choisis pour préparer un composé de formule (I) ou un sel de celui-ci où $-NR^3R^4$ représente un groupe morpholino, un groupe pipérazinyle, un groupe pipérazinyle méthyl-substitué, un groupe 1-pyrrolidinyle ou un groupe pipéridyle.

10. Procédé selon la revendication 9, dans lequel les composés réagissants et les conditions de réaction sont choisis pour préparer un composé de formule (I) ou un sel de celui-ci où $-NR^3R^4$ représente un groupe morpholino, un groupe 1-pipérazinyle, un groupe 4-méthyl-1-pipérazinyle, un groupe 1-pyrrolidinyle ou un groupe pipéridino.

11. Procédé selon l'une quelconque des revendications 1 et 7 à 10, dans lequel les composés réagissants et les conditions de réaction sont choisis pour préparer un composé de formule (I) ou un sel de celui-ci où A représente un groupe alkyle ayant de 1 à 4 atomes de carbone, un groupe alcoxy ayant de 1 à 4 atomes de carbone ou un groupe phényle, phénoxy, benzyle ou benzyloxy non substitués.

12. Procédé selon la revendication 11, dans lequel les composés réagissants et les conditions de réaction sont choisis pour préparer un composé de formule (I) ou un sel de celui-ci où A représente un groupe méthyle, un groupe méthoxy ou un groupe phényle, phénoxy, benzyle ou benzyloxy non substitués.

13. Procédé selon la revendication 1, dans lequel les composés réagissants et les conditions de réaction sont choisis pour préparer un composé de formule (I) ou un sel de celui-ci où :
$R^1$ représente un atome d'hydrogène, un atome d'halogène ou un groupe alkyle ayant de 1 à 4 atomes de carbone ;
$R^2$ représente un groupe phényle ou un groupe phényle ayant un substituant halogène ;
$-NR^3R^4$ représente un groupe morpholino, pipéridyle ou pipérazinyle ; et
A représente un groupe alkyle ou alcoxy ayant de 1 à 4 atomes de carbone.

14. Procédé selon la revendication 1, dans lequel les composés réagissants et les conditions de réaction sont choisis pour préparer un composé de formule (I) ou un sel de celui-ci où :
$R^1$ représente un atome d'hydrogène, un atome d'halogène ou un groupe alkyle ayant de 1 à 4 atomes de carbone ;
$R^2$ représente un groupe phényle ou un groupe phényle ayant un seul substituant halogène ;
$-NR^3R^4$ représente un groupe morpholino ; et
A représente un groupe alcoxy ayant de 1 à 4 atomes de carbone.

15. Procédé selon la revendication 14, dans lequel les composés réagissants et les conditions de réaction sont choisis pour préparer un composé de formule (I) ou un sel de celui-ci où $R^1$ représente un atome d'hydrogène, un atome de chlore ou un groupe méthyle ou éthyle.

16. Procédé selon la revendication 1, dans lequel les composés réagissants et les conditions de réaction sont choisis pour préparer :
la 2-(2-acétoxy-3-morpholinopropyl)-5-(p-chlorophényl)-3-isoxazolone ;
la 5-(p-chlorophényl)-2-(2-propionyloxy-3-morpholinopropyl)-3-isoxazolone ;
la 2-(2-méthoxycarbonyloxy-3-morpholinopropyl)-5-phényl-3-isoxazolone ;
la 2-(2-éthoxycarbonyloxy-3-morpholinopropyl)-5-phényl-3-isoxazolone ;
la 4-chloro-2-(2-méthoxycarbonyloxy-3-morpholinopropyl)-5-phényl-3-isoxazolone ;
la 4-chloro-2-(2-éthoxycarbonyloxy-3-morpholinopropyl)-5-phényl-3-isoxazolone ;
la 2-(2-méthoxycarbonyloxy-3-morpholinopropyl)-4-méthyl-5-phényl-3-isoxazolone ;
la 4-éthyl-2-(2-méthoxycarbonyloxy-3-morpholinopropyl)-5-phényl-3-isoxazolone ;
la 5-(p-fluorophényl)-2-(2-méthoxycarboxy-3-morpholinopropyl)-3-isoxazolone ;
ou un de leurs sels pharmaceutiquement acceptables.

17. Procédé selon l'une quelconque des revendications 1 à 16, dans lequel le dérivé réactif dudit acide carboxylique de formule (III) est l'halogénure de formule (IV) :

$$X\text{-}CO\text{-}A \qquad (IV)$$

(dans laquelle A est tel que défini dans la revendication 1 et X représente un atome d'halogène).

18. Procédé pour préparer une composition pharmaceutique pour le traitement des troubles cérébro-vasculaires ou comme myorelaxant à action centrale, qui comprend le mélange d'un composé de formule (I)

ou d'un sel d'addition d'acide pharmaceutiquement acceptable de celui-ci selon l'une quelconque des revendications 1 à 16, avec un véhicule ou diluant pharmaceutiquement acceptables.

**19.** Utilisation d'au moins un composé selon l'une quelconque des revendications 1 à 16, pour le traitement des troubles cérébro-vasculaires, pour l'utilisation comme myorelaxant à action centrale ou pour l'utilisation comme antidépresseur.

**20.** Utilisation d'au moins un composé selon l'une quelconque des revendications 1 à 16, pour la préparation d'un médicament pour le traitement des troubles cérébro-vasculaires, pour l'utilisation comme myorelaxant à action centrale ou pour l'utilisation comme antidépresseur.